(19) Europäisches Patentamt
European Patent Office
Office européen des brevets

(11) EP 0 637 297 B1

(12) EUROPEAN PATENT SPECIFICATION

(45) Date of publication and mention of the grant of the patent:
**23.08.2000 Bulletin 2000/34**

(21) Application number: **93910835.3**

(22) Date of filing: **22.04.1993**

(51) Int. Cl.$^7$: **C07C 63/49**, A61K 31/19, A61K 31/44, C07C 63/72, C07C 65/40, C07C 69/76, C07C 235/84, C07D 307/68, C07D 333/38, C07D 213/80, C07D 311/58, C07D 213/79, C07D 207/40

(86) International application number:
**PCT/US93/03944**

(87) International publication number:
**WO 93/21146 (28.10.1993 Gazette 1993/26)**

(54) **COMPOUNDS HAVING SELECTIVITY FOR RETINOID X RECEPTORS**

RETINOID-X REZEPTOR SELEKTIVE VERBINDUNGEN

COMPOSES PRESENTANT UNE ACTIVITE SELECTIVE PAR RAPPORT A DES RECEPTEURS DE RETINOIDE X

(84) Designated Contracting States:
**AT BE CH DE DK ES FR GB GR IE IT LI LU MC NL PT SE**

(30) Priority: **22.04.1992 US 872707**
**11.09.1992 US 944783**
**11.01.1993 US 3223**
**05.03.1993 US 27747**
**21.04.1993 US 52051**

(43) Date of publication of application:
**08.02.1995 Bulletin 1995/06**

(60) Divisional application:
**99118828.5 / 0 983 992**
**99118827.7 / 0 983 991**

(73) Proprietor:
**LIGAND PHARMACEUTICALS, INC.**
**San Diego California 92121-1117 (US)**

(72) Inventors:
- **BOEHM, Marcus F.**
**San Diego, CA 92129 (US)**
- **HEYMAN, Richard A.**
**Encinitas, CA 92024 (US)**
- **ZHI, Lin**
**California 92129 (US)**

(74) Representative:
**Sexton, Jane Helen et al**
**J.A. KEMP & CO.**
**14 South Square**
**Gray's Inn**
**London WC1R 5LX (GB)**

(56) References cited:

| | |
|---|---|
| **EP-A- 0 260 162** | **EP-A- 0 284 261** |
| **WO-A-85/00806** | **WO-A-93/06086** |
| **WO-A-94/12880** | **DE-A- 3 711 546** |
| **FR-A- 1 444 545** | **FR-A- 2 307 524** |
| **FR-A- 2 601 670** | **GB-A- 2 197 316** |
| **US-A- 4 548 723** | |

- **CHEMICAL ABSTRACTS, vol. 83, no. 6, 11 August 1975 Columbus, Ohio, US; abstract no. 44730f, Y. MIYAZAWA ET AL. 'Pyridinecarboxylic acid lactones color formers' page 72; & DE-A-24 35 408 (HODOGAYA CHEMICAL CO., LTD.)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 637 297 B1

## Description

This invention relates to intracellular receptors and ligands therefor. More specifically, this invention relates to compounds having selective activity for specific retinoic acid receptors, and methods for use of such compounds.

The vitamin A metabolite retinoic acid has long been recognized to induce a broad spectrum of biological effects. A variety of structural analogues of retinoic acid have been synthesized that also have been found to be bioactive. Some, such as Retin-A® (registered trademark of Johnson & Johnson) and Accutane® (registered trademark of Hoffmann-LaRoche), have found utility as therapeutic agents for the treatment of various pathological conditions. Metabolites of vitamin A and their synthetic analogues are collectively herein called "retinoids".

Synthetic retinoids have been found to mimic many of the pharmacological actions of retinoic acid. However, the broad spectrum of pharmacological actions of retinoic acid is not reproduced in full by all bioactive synthetic retinoids.

Medical professionals have become very interested in the medicinal applications of retinoids. Among their uses approved by the FDA is the treatment of severe forms of acne and psoriasis. A large body of evidence also exists that these compounds can be used to arrest and, to an extent, reverse the effects of skin damage arising from prolonged exposure to the sun. Other evidence exists that these compounds may be useful in the treatments of a variety of severe cancers including melanoma, cervical cancer, some forms of leukemia, and basal and squamous cell carcinomas. Retinoids have also shown an ability to be efficacious in treating premalignant cell lesions, such as oral leukoplakia, and to prevent the occurrence of malignancy.

Use of the retinoids is associated with a number of significant side effects. The most serious of these is that, as a class, they are among the most potent teratogens known. Teratogens are compounds that cause severe birth defects during specific periods of fetal exposure. Other side effects include irritation of the tissues treated, which can be so severe that patients cannot tolerate treatment.

Various investigations have been undertaken to elucidate the structure-activity relationships governing the abilities of synthetic retinoids to induce the various pharmacological consequences of retinoic acid exposure. This has been a complicated task, however, since the assays available to investigators have been bioassays, carried out either in intact animals or in isolated tissues. Technical constraints have often dictated the use of different small animal species for different assays. Interpretation of results has been complicated by possible pharmacokinetic and metabolic effects and possible species differences in the receptors involved. Nevertheless, definite differences in the pharmacological effects of various synthetic retinoids have been observed.

Major insight into the molecular mechanism of retinoic acid signal transduction was gained in 1988. Prior to that time, several high abundance cellular retinoid binding proteins were incorrectly inferred to be the signal transducing receptors for retinoic acid. In 1988, a member of the steroid/thyroid hormone intracellular receptor superfamily (Evans, *Science,* 240:889-95 (1988)) was shown to transduce a retinoic acid signal (Giguere et al., *Nature,* 330:624-29 (1987); Petkovich *et al., Nature,* 330: 444-50 (1987)). This unexpected finding related retinoic acid to other non-peptide hormones and elucidated the mechanism of retinoic acid effects in altering cell function. It is now known that retinoids regulate the activity of two distinct intracellular receptor sub-families; the Retinoic Acid Receptors (RARs) and the Retinoid X Receptors (RXRs).

The first retinoic acid receptor identified, designated RAR-alpha, acts to modulate transcription of specific target genes in a manner which is ligand-dependent, as has been shown to be the case for many of the members of the steroid/thyroid hormone intracellular receptor superfamily. The endogenous low-molecular-weight ligand upon which the transcription-modulating activity of RAR-alpha depends is all-*trans*-retinoic acid. Retinoic acid receptor-mediated changes in gene expression result in characteristic alterations in cellular phenotype, with consequences in many tissues manifesting the biological response to retinoic acid. Two additional genes closely related to RAR-alpha were recently identified and were designated PAR-beta and PAR-gamma and are very highly related (Brand *et al., Nature*, 332:850-53 (1988); Ishikawa *et al., Mol. Endocrin.*, 4:837-44 (1990)). In the region of the retinoid receptors which can be shown to confer ligand binding, the primary amino acid sequences diverge by less than 15% among the three PAR subtypes or isoforms. All-*trans*-retinoic acid is a natural ligand for the retinoic acid receptors (RARs) and is capable of binding to these receptors with high affinity, resulting in the regulation of gene expression. The newly-discovered retinoid metabolite, 9-*cis*-retinoic acid, is also an activator of RARs.

A related but unexpected observation was made recently (Mangelsdorf *et al., Nature,* 345:224-29 (1990)), in which another member of the steroid/thyroid receptor superfamily was also shown to be responsive to retinoic acid. This new retinoid receptor subtype has been designated Retinoid X Receptor (RXR), because certain earlier data suggested that a derivative of all-*trans*-retinoic acid may be the endogenous ligand for RXR. Like the RARs, the RXRs are also known to have at least three subtypes or isoforms, namely RXR-alpha, RXR-beta, and RXR-gamma, with corresponding unique patterns of expression (Manglesdorf et al., *Genes & Devel.*, 6:329-44 (1992)).

Although both the RARs and RXRs respond to all-*trans*-retinoic acid *in vivo*, the receptors differ in several important aspects. First, the RARs and RXRs are significantly divergent in primary structure (*e.g.*, the ligand binding

domains of RARα and RXRα have only 27% amino acid identity). These structural differences are reflected in the different relative degrees of responsiveness of RARs and RXRs to various vitamin A metabolites and synthetic retinoids. In addition, distinctly different patterns of tissue distribution are seen for RARs and RXRs. For example, in contrast to the RARs, which are not expressed at high levels in the visceral tissues, RXAα mRNA has been shown to be most abundant in the liver, kidney, lung, muscle and intestine. Finally, the RARs and RXRs have different target gene specificity. For example, response elements have recently been identified in the cellular retinal binding protein type II (CRBPII) and apolipoprotein AI genes which confer responsiveness to RXR, but not RAR. Furthermore, RAR has also been recently shown to repress RXR-mediated activation through the CRBPII RXR response element (Manglesdorf *et al.*, *Cell*, 66:555-61 (1991)). These data indicate that two retinoic acid responsive pathways are not simply redundant, but instead manifest a complex interplay. Recently, Heyman *et al*. (*Cell*, 68:397-406 (1992)) and Levin *et al.* (*Nature,* 355:359-61 (1992)) independently demonstrated that 9-*cis*-retinoic acid is a natural endogenous ligand for the RXRs. 9-*cis*-retinoic acid was shown to bind and transactivate the RXRs, as well as the RARs, and therefore appears to act as a "bifunctional" ligand.

In view of the related, but clearly distinct, nature of these receptors, ligands which are more selective for the Retinoid X Receptor subfamily would be of great value for selectively controlling processes mediated by one or more of the RXR isoforms, and would provide the capacity for independent control of the physiologic processes mediated by the RXRs. Ligands which preferentially affect one or more but not all of the receptor isoforms also offer the possibility of increased therapeutic efficacy when used for medicinal applications.

The entire disclosures of the publications and references referred to above and hereafter in this specification are incorporated herein by reference.

## Summary of the Invention

The present invention is directed to compounds, compositions, and methods for modulating processes mediated by one or more Retinoid X Receptors. More particularly, the invention relates to compounds which selectively or preferentially activate Retinoid X Receptors, in comparison to Retinoic Acid Receptors. These compounds selectively modulate processes mediated by Retinoid X Receptors. Accordingly, the invention also relates to methods for modulating processes selectively mediated by one or more Retinoid X Receptors, in comparison to Retinoic Acid Receptors, by use of the compounds of this invention. Examples of compounds used in and forming part of the invention include bicyclic benzyl, pyridinyl, thiophene, furanyl, and pyrrole derivatives. Pharmaceutical compositions containing the compounds disclosed are also within the scope of this invention. Also included are methods for identifying or purifying Retinoid X Receptors by use of the compounds of this invention.

## Brief Description of the Figures

The present invention may be better understood and its advantages appreciated by those skilled in the art by referring to the accompanying drawings wherein:

Figure 1 presents the standardized dose response profiles showing the transactivation of RAR and RXR isoforms by 3-methyl-TTNCB.
Figure 2 presents the standardized dose response profiles showing the transactivation of RAR and RXR isoforms by all-*trans*-retinoic acid.
Figure 3 presents the standardized dose response profiles showing the transactivation of RAR and RXR isoforms by 9-*cis*-retinoic acid.
Figure 4 presents the standardized dose response profiles showing the transactivation of RAR and RXR isoforms by 3-methyl-TTNEB.
Figure 5 presents the standardized dose response profiles showing the transactivation of RAR and RXR isoforms by 3-bromo-TTNEB.
Figure 6 presents the standardized dose response profiles showing the transactivation of RAR and RXR isoforms by 3-methyl-TTNCHBP.
Figure 7 presents the standardized dose response profiles showing the transactivation of RAR and RXR isoforms by 3-methyl-TTNEHBP.
Figure 8 presents the inhibition of transglutaminase activity by 9-*cis*-retinoic acid, all-*trans*-retinoic acid, and 3-methyl-TTNCB.
Figure 9 presents the Topical Dose Response, based on the test on Rhino mice, for 9-*cis*-retinoic acid, all-*trans*-retinoic acid, 3-methyl-TTNCB, 1, 25-dihydroxy Vitamin D.
Figure 10 presents the effect on rat HDL cholesterol of all-*trans*-retinoic acid, 9-*cis*-retinoic acid, 3-methyl-TTNCB, and 3-methyl-TTNEB.

Figure 11 presents the concentration-related effect of 3-methyl-TTNEB and TTNPB individually on incorporation of radiolabeled thymidine into DNA.

Figure 12 presents the concentration-related effect of a combination of 3-methyl-TTNEB and TTNPB on incorporation of radiolabeled thymidine into DNA.

This invention discloses retinoid-like compounds or ligands which have selective activity for members of the subfamily of Retinoid X Receptors (RXRs), in comparison to members of the subfamily of Retinoic Acid Receptors (RARs). Examples of such compounds are bicyclic benzyl derivatives which can be represented by the formulae:

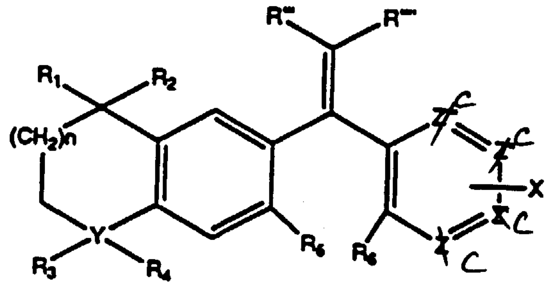

or

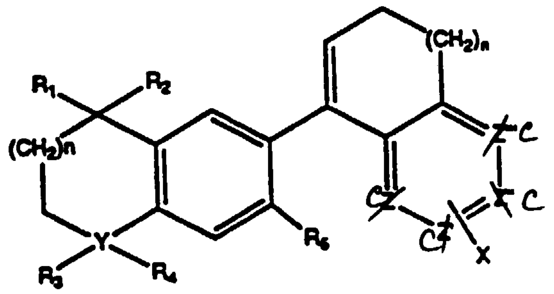

or

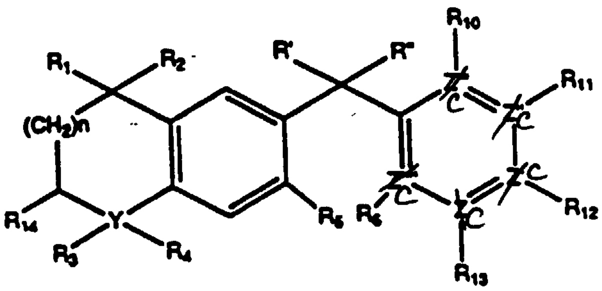

wherein

$R_1$ and $R_2$, each independently, represent hydrogen or lower alkyl or acyl having 1-4 carbon atoms;
Y represents C, O, S, N, CHOH, CO, SO, $SO_2$, or a pharmaceutically acceptable salt;
$R_3$ represents hydrogen or lower alkyl having 1-4 carbon atoms where Y is C or N;
$R_4$ represents hydrogen or lower alkyl having 1-4 carbon atoms where Y is C, but $R_4$ does not exist if Y is N, and neither $R_3$ or $R_4$ exist if Y is S, O, CHOH, CO, SO, or $SO_2$;
R' and R'' represent hydrogen, lower alkyl or acyl having 1-4 carbon atoms, OH, alkoxy having 1-4 carbon atoms, thiol or thio ether, or amino,
or R' and R'' taken together form an oxo (keto), methano, thioketo, epoxy, cyclopropyl, or cycloalkyl group and wherein the epoxy, cyclopropyl, and cycloalkyl groups can be substituted with lower alkyl having 1-4 carbons or halogen;
R''' and R'''' represent hydrogen, halogen, lower alkyl or acyl having 1-4 carbon atoms,
or R''' and R'''' taken together form a cycloalkyl group having 3-10 carbons, and wherein the cycloalkyl group can be substituted with lower alkyl having 1-4 carbons or halogen;
$R_5$ represents a lower alkyl having 1-4 carbons, halogen, nitro, $OR_7$, $SR_7$, $NR_7R_8$, or $(CF_2)_nCF_3$;
$R_6$, $R_{10}$, $R_{11}$, $R_{12}$, $R_{13}$ each independently represent hydrogen, a lower alkyl having 1-4 carbons, halogen, nitro,

$OR_7$, $SR_7$, $NR_7R_8$ or $(CF_2)_nCF_3$, with the proviso that one of $R_{11}$, $R_{12}$ or $R_{13}$ is X;
$R_7$ and $R_8$ each individually represent hydrogen or a lower alkyl having 1-6 carbons;
$R_{14}$ represents hydrogen, a lower alkyl having 1-4 carbons, oxo, hydroxy, acyl having 1-4 carbons, halogen, thiol, or thioketone;
X is COOH, tetrazole, $PO_3H$, $SO_3H$, CHO, $CH_2OH$, $CONH_2$, COSH, $COOR_9$, $COSR_9$, $CONHR_9$, or COOW where $R_9$ represents a lower alkyl having 1-4 carbons, phenyl, aromatic alkyl, or q-hydroxyphenyl, q-bromophenyl, q-chlorophenyl, q-fluorophenyl, or q-iodophenyl, where q=2-4, where W is a pharmaceutically acceptable salt, and where X can originate from any C except the 2 position on the ring;
n = 0-3.

As used in this disclosure, pharmaceutically acceptable salts include but are not limited to: hydrochloric, hydrobromic, hydroiodic, hydrofluoric, sulfuric, citric, maleic, acetic, lactic, nicotinic, succinic, oxalic, phosphoric, malonic, salicylic, phenylacetic, stearic, pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and tris(hydroxymethyl)aminomethane. Additional pharmaceutically acceptable salts are known to those of skill in the art.

Representative derivatives according to the present invention include the following:

p[3,5,5,8,8-pentamethyl-1,2,3,4-tetrahydro-2-naphthyl-(2-carbonyl)]-benzoic acid, also known as 4-[(3,5,5,8,8-pentamethyl-5,6,7,8-tetrahydro-2-naphthyl) carbonyl]benzoic acid, and designated "3-methyl-TTNCB";
p[5,5,8,8-tetramethyl-1,2,3,4-tetrahydro-3-isopropyl-2-naphthyl-(2-carbonyl)]-benzoic acid, also known as 4-[(3-isopropyl-5,5,8,8-tetramethyl-5,6,7,8-tetrahydro-2-naphthyl)carbonyl]benzoic acid, and designated "3-IPR-TTNCB" or Compound 37;
p[5,5,8,8-tetramethyl-1,2,3,4-tetrahydro-3-isopropyl-2-naphthyl-(2-methano)]-benzoic acid, also known as 4-[1-(3-isopropyl-5,5,8,8-tetramethyl-5,6,7,8-tetrahydro-2-naphthyl)ethenyl]benzoic acid, and designated "3-IPR-TTNEB" or Compound 42;
p[5,5,8,8-tetramethyl-1,2,3,4-tetrahydro-3-ethyl-2-naphthyl-(2-methano)]-benzoic acid, also known as 4-[1-(3-ethyl-5,5,8,8-tetramethyl-5,6,7,8-tetrahydro-2-naphthyl)ethenyl]benzoic acid, and designated "3-ethyl-TTNEB" or Compound 45;
p[5,5,8,8-tetramethyl-1,2,3,4-tetrahydro-3-bromo-2-naphthyl-(2-methano)]-benzoic acid, also known as 4-[1-(3-bromo-5,5,8,8-tetramethyl-5,6,7,8-tetrahydro-2-naphthyl)ethenyl]benzoic acid, and designated "3-bromo-TTNEB" or Compound 46;
p[5,5,8,8-tetramethyl-1,2,3,4-tetrahydro-3-chloro-2-naphthyl-(2-methano)]-benzoic acid, also known as 4-[1-(3-chloro-5,5,8,8-tetramethyl-5,6,7,8-tetrahydro-2-naphthyl)ethenyl]benzoic acid, and designated "3-chloro-TTNEB" or Compound 43;
p[3,5,5,8,8-pentamethyl-1,2,3,4-tetrahydro-2-naphthyl-(2-methano)]-benzoic acid, also known as 4-[1-(3,5,5,8,8-pentamethyl-5,6,7,8-tetrahydro-2-naphthyl)ethenyl]benzoic acid, and designated "3-methyl-TTNEB";
p[3,5,5,8,8-pentamethyl-1,2,3,4-tetrahydro-2-naphthyl-(2-hydroxymethyl)]-benzoic acid, also known as 4-[1-(3,5,5,8,8-pentamethyl-5,6,7,8-tetrahydro-2-naphthyl)hydroxymethyl]benzoic acid, and designated "3-methyl-TTNHMB";
p[5,5,8,8-tetramethyl-1,2,3,4-tetrahydro-3-bromo-2-naphthyl-(2-carbonyl)]-benzoic acid, also known as 4-[(3-bromo-5,5,8,8-tetramethyl-5,6,7,8-tetrahydro-2-naphthyl)carbonyl]benzoic acid, and designated "3-bromo-TTNCB" or Compound 41;
p[5,5,8,8-tetramethyl-1,2,3,4-tetrahydro-3-chloro-2-naphthyl-(2-carbonyl)]-benzoic acid, also known as 4-[(3-chloro-5,5,8,8-tetramethyl-5,6,7,8-tetrahydro-2-naphthyl)carbonyl]benzoic acid, and designated "3-chloro-TTNCB" or Compound 38;
p[5,5,8,8-tetramethyl-1,2,3,4-tetrahydro-3-hydroxy-2-naphthyl-(2-carbonyl)]-benzoic acid, also known as 4-[(3-hydroxy-5,5,8,8-tetramethyl-5,6,7,8-tetrahydro-2-naphthyl)carbonyl]benzoic acid, and designated "3-hydroxy-TTNCB" or Compound 39;
p[5,5,8,8-tetramethyl-1,2,3,4-tetrahydro-3-ethyl-2-naphthyl-(2-carbonyl)]-benzoic acid, also known as 4-[(3-ethyl-5,5,8,8-tetramethyl-5,6,7,8-tetrahydro-2-naphthyl)carbonyl]benzoic acid, and designated "3-ethyl-TTNCB" or Compound 40;
p[3,5,5,8,8-pentamethyl-1,2,3,4-tetrahydro-2-naphthyl-(2-thioketo)]-benzoic acid, also known as 4-[(3,5,5,8,8-pentamethyl-5,6,7,8-tetrahydro-2-naphthyl) thioketo]benzoic acid, and designated "thioketone";
p[3,5,5,8,8-pentamethyl-1,2,3,4-tetrahydro-2-naphthyl-(2-carbonyl)]-N-(4-hydroxyphenyl)benzamide, also known as 4-[(3,5,5,8,8-pentamethyl-5,6,7,8-tetrahydro-2-naphthyl)carbonyl]-N-(4-hydroxyphenyl)benzamide, and designated "3-methyl-TTNCHBP";
p[3,5,5,8,8-pentamethyl-1,2,3,4-tetrahydro-2-naphthyl-(2-methano)]-N-(4-hydroxyphenyl)benzamide, also known

as 4-[1-(3,5,5,8,8-pentamethyl-5,6,7,8-tetrahydro-2-naphthyl)ethenyl]-N-(4-hydroxyphenyl)benzamide, and designated "3-methyl-TTNEHBP" or Compound 63;

4-[1-(3,5,5,8,8-pentamethyl-5,6,7,8-tetrahydro-2-naphthyl) epoxy]benzoic acid, designated "TPNEB" or Compound 47;

4-[1-(3,5,5,8,8-pentamethyl-5,6,7,8-tetrahydro-2-naphthyl) cyclopropyl]benzoic acid, designated "TPNCB" or Compound 48; and

4-[1-(3,5,5,8,8-pentamethyl-5,6,7,8-tetrahydro-2-naphthyl)ethenyl]benzenetetrazole, designated "3-methyl-TTNEBT" or Compound 55;

Representative structures for such compounds are as follows:

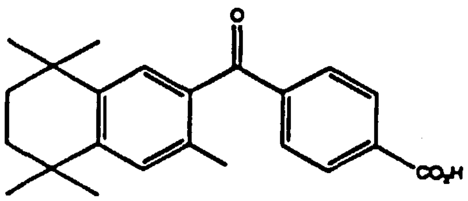

3-methyl-TTNCB

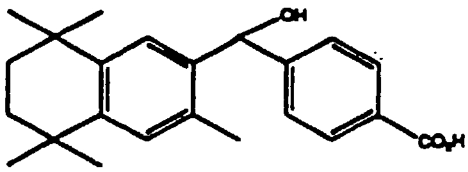

3-methyl-TTNHMB

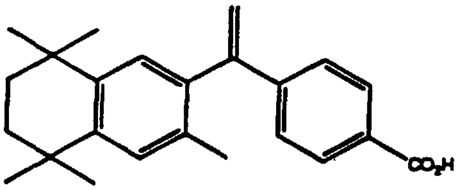

3-methyl-TTNEB

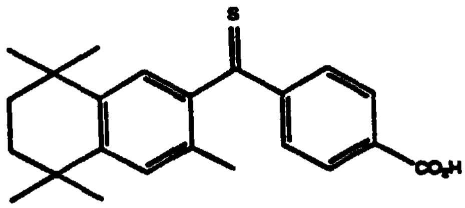

thioketone

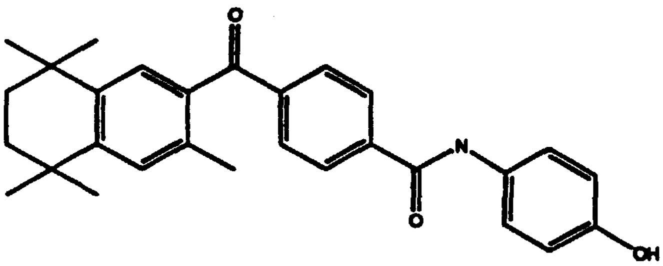

3-methyl-TTNCHBP

37
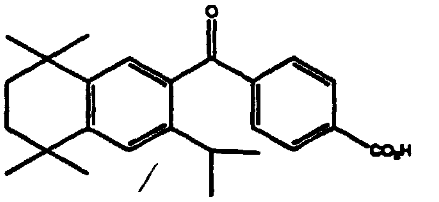

4-[(3-isopropyl-5,5,8,8-tetramethyl-5,6,7,8-tetrahydro-2-naphthyl)carbonyl] benzoic acid (3-IPr-TTNCB)

38
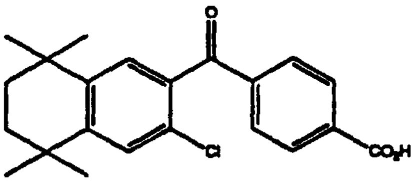

4-[(3-chloro-5,5,8,8-tetramethyl-5,6,7,8-tetrahydro-2-naphthyl)carbonyl] benzoic acid (3-chloro-TTNCB)

39
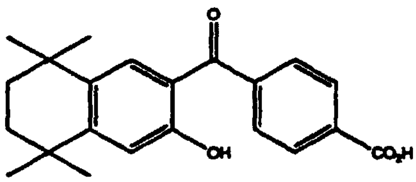

4-[(3-hydroxy-5,5,8,8-tetramethyl-5,6,7,8-tetrahydro-2-naphthyl)carbonyl] benzoic acid (3-hydroxy-TTNCB)

40
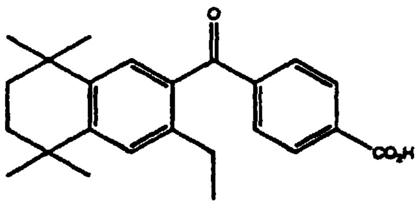

4-[(3-ethyl-5,5,8,8-tetramethyl-5,6,7,8-tetrahydro-2-naphthyl)carbonyl] benzoic acid (3-Et-TTNCB)

41
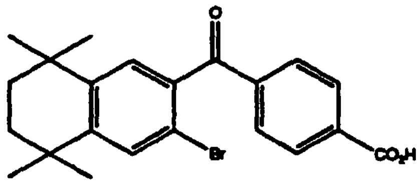

4-[(3-bromo-5,5,8,8-tetramethyl-5,6,7,8-tetrahydro-2-naphthyl)carbonyl] benzoic acid (3-bromo-TTNCB)

42
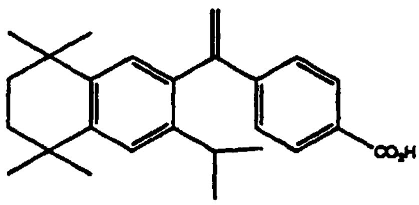

4-[1-(3-isopropyl-5,5,8,8-tetramethyl-5,6,7,8-tetrahydro-2-naphthyl)ethenyl] benzoic acid (3-IPr-TTNEB)

43
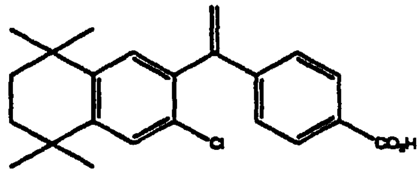

4-[1-(3-chloro-5,5,8,8-tetramethyl-5,6,7,8-tetrahydro-2-naphthyl)ethenyl] benzoic acid (3-chloro-TTNEB)

44
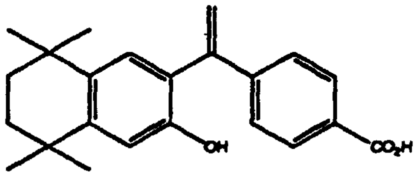

4-[1-(3-hydroxy-5,5,8,8-tetramethyl-5,6,7,8-tetrahydro-2-naphthyl) ethenyl] benzoic acid (3-hydroxy-TTNEB)

45
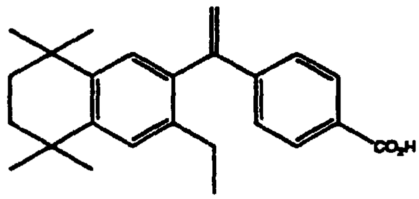

4-[1-(3-ethyl-5,5,8,8-tetramethyl-5,6,7,8-tetrahydro-2-naphthyl)ethenyl] benzoic acid (3-Et-TTNEB)

46
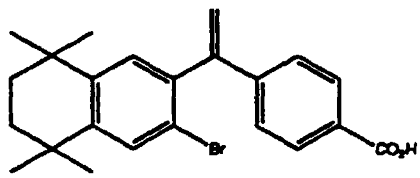

4-[1-(3-bromo-5,5,8,8-tetramethyl-5,6,7,8-tetrahydro-2-naphthyl)ethenyl] benzoic acid (3-bromo-TTNEB)

47
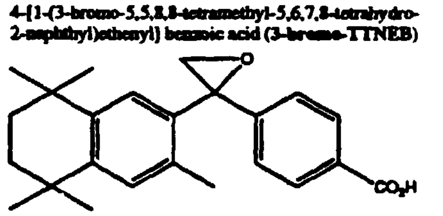

4-[1-(3,5,5,8,8-pentamethyl-5,6,7,8-tetrahydro-2-naphthyl)epoxy] benzoic acid (TPNEB)

48
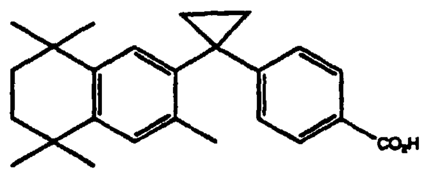

4-[1-(3,5,5,8,8-pentamethyl-5,6,7,8-tetrahydro-2-naphthyl)cyclopropyl] benzoic acid (TPNCB)

49

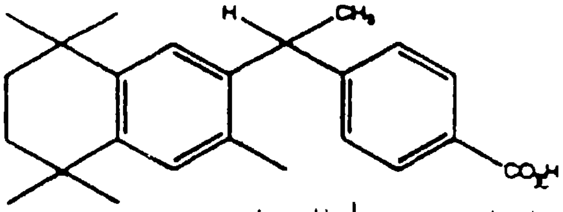

4-[1-(3,5,5,8,8-pentamethyl-5,6,7,8-tetrahydro-2-naphthyl)ethyl] benzoic acid (PTNEB)

50

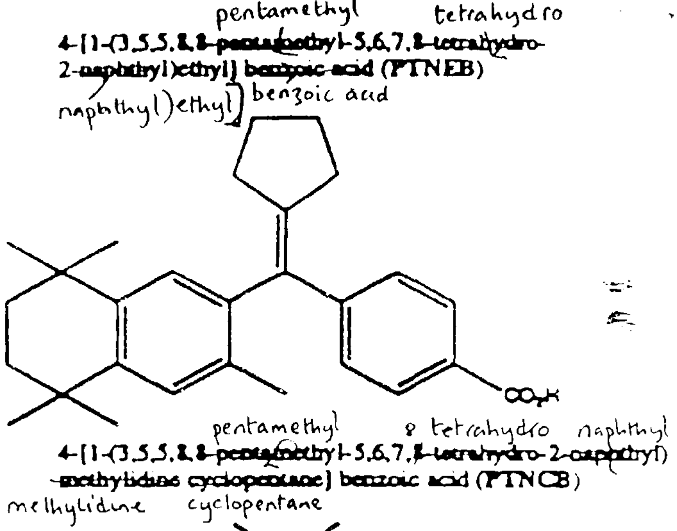

4-[1-(3,5,5,8,8-pentamethyl-5,6,7,8-tetrahydro-2-naphthyl)-methylidene cyclopentane] benzoic acid (PTNCB)

51

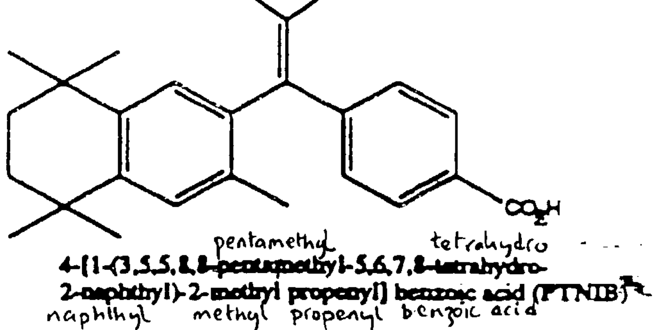

4-[1-(3,5,5,8,8-pentamethyl-5,6,7,8-tetrahydro-2-naphthyl)-2-methyl propenyl] benzoic acid (PTNIB)

54

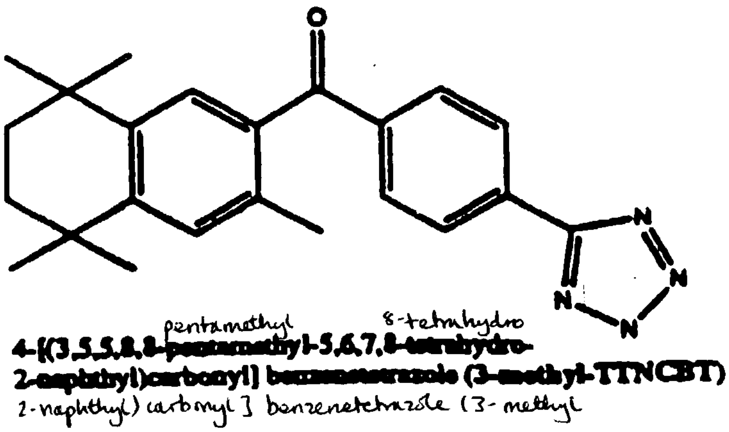

4-[(3,5,5,8,8-pentamethyl-5,6,7,8-tetrahydro-2-naphthyl)carbonyl] benzenetetrazole (3-methyl-TTNCBT)

55

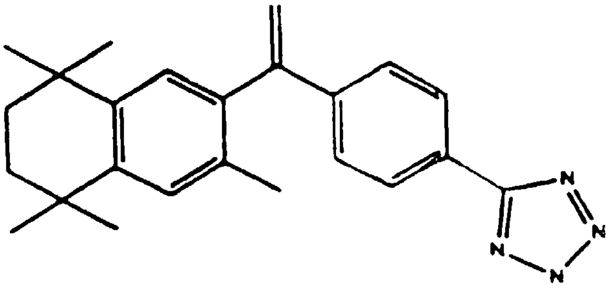

4-[1-(3,5,5,8,8-pentamethyl-5,6,7,8-tetrahydro-
2-naphthyl)ethenyl] benzenetetrazole (3-methyl-TTNEBT)

2-naphthyl)ethenyl] benzenetetrazole (3-methyl - TTNEBT

63

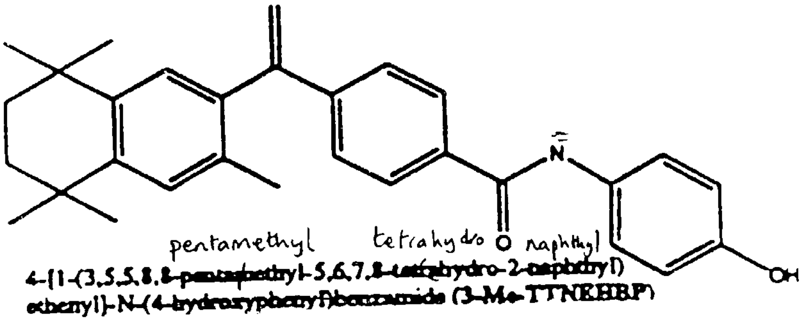

pentamethyl tetrahydro naphthyl

4-[1-(3,5,5,8,8-pentamethyl-5,6,7,8-tetrahydro-2-naphthyl)
ethenyl]-N-(4-hydroxyphenyl)benzamide (3-Me-TTNEHBP)

ethenyl (4-hydroxyphenyl)benzamide

64

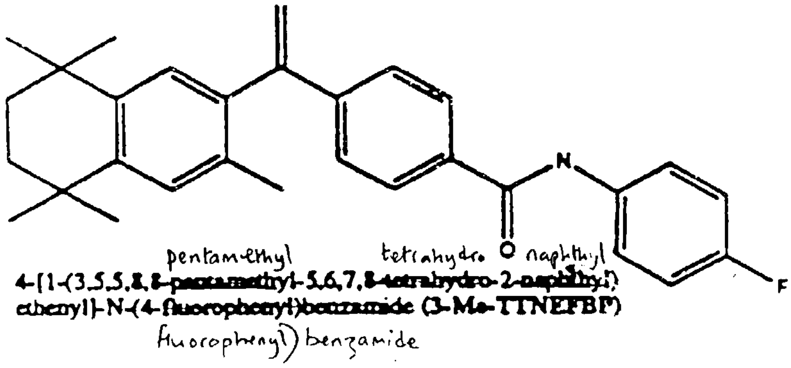

pentamethyl tetrahydro naphthyl

4-[1-(3,5,5,8,8-pentamethyl-5,6,7,8-tetrahydro-2-naphthyl)
ethenyl]-N-(4-fluorophenyl)benzamide (3-Me-TTNEFBP)

fluorophenyl) benzamide

65

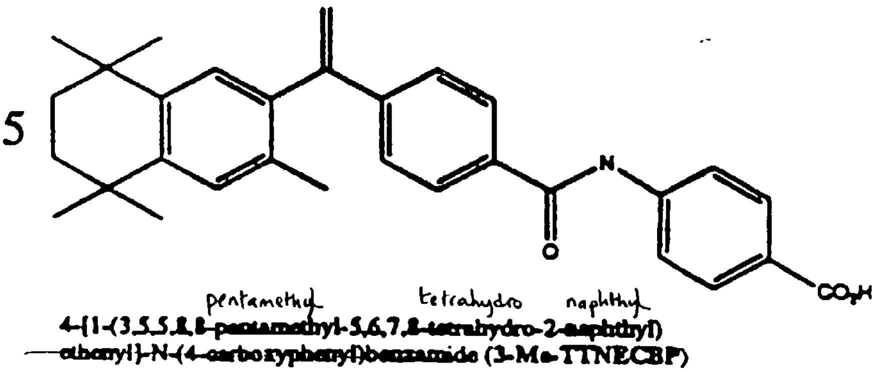

pentamethyl tetrahydro naphthyl

4-[1-(3,5,5,8,8-pentamethyl-5,6,7,8-tetrahydro-2-naphthyl)
ethenyl]-N-(4-carboxyphenyl)benzamide (3-Me-TTNECBP)

ethenyl]-N-(4-carboxyphenyl)benzamide

66 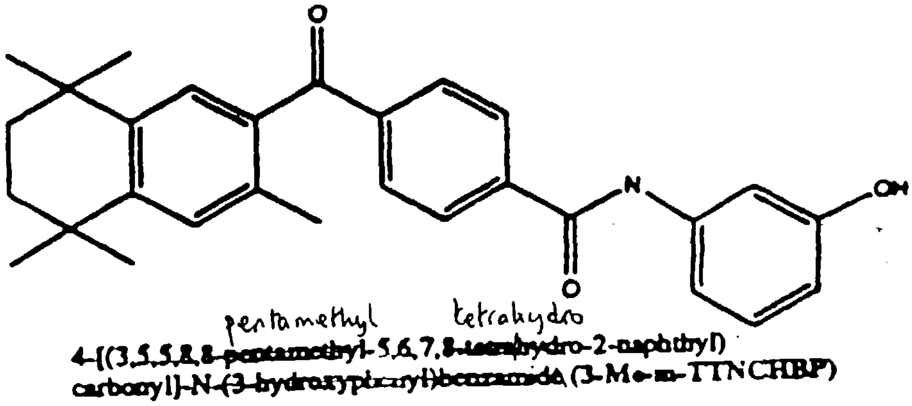

pentamethyl tetrahydro

4-[(3,5,5,8,8-pentamethyl-5,6,7,8-tetrahydro-2-naphthyl) carbonyl]-N-(3-hydroxyphenyl)benzamide (3-Me-m-TTNCHBP)

carbonyl]-N-(3-hydroxyphenyl)benzamide

67 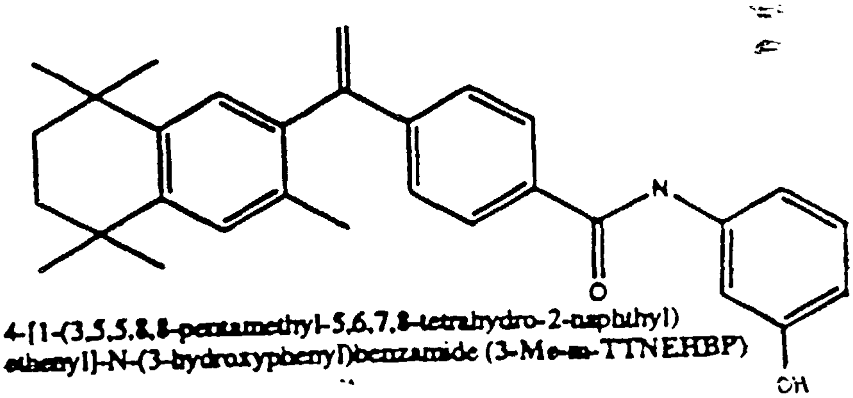

4-[1-(3,5,5,8,8-pentamethyl-5,6,7,8-tetrahydro-2-naphthyl) ethenyl]-N-(3-hydroxyphenyl)benzamide (3-Me-m-TTNEHBP)

ethenyl

68 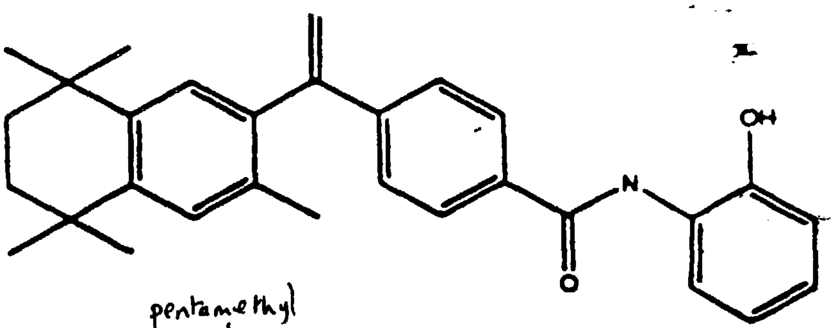

pentamethyl

4-[1-(3,5,5,8,8-pentamethyl-5,6,7,8-tetrahydro-2-naphthyl) ethenyl]-N-(2-hydroxyphenyl)benzamide(3-Me-o-TTNCHBP)

ethenyl (2-hydroxyphenyl)benzamide

69

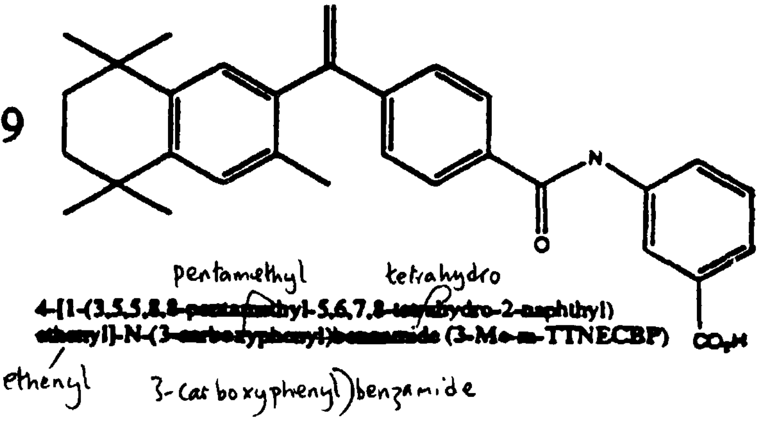

4-[1-(3,5,5,8,8-pentamethyl-5,6,7,8-tetrahydro-2-naphthyl) ethenyl]-N-(3-carboxyphenyl)benzamide (3-Me-m-TTNECBP)

70

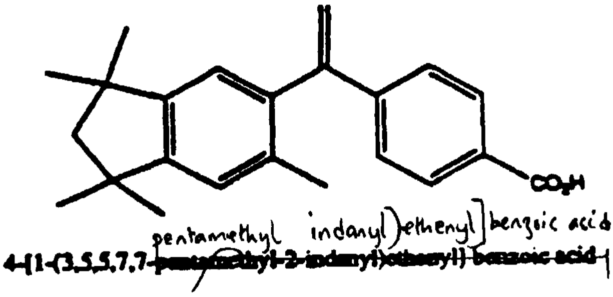

4-[1-(3,5,5,7,7-pentamethyl-2-indanyl)ethenyl] benzoic acid

71

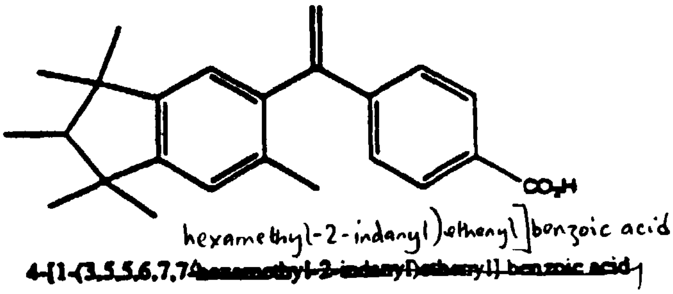

4-[1-(3,5,5,6,7,7-hexamethyl-2-indanyl)ethenyl] benzoic acid

72

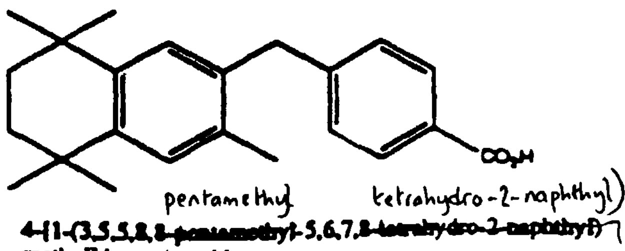

4-[1-(3,5,5,8,8-pentamethyl-5,6,7,8-tetrahydro-2-naphthyl) -methyl] benzoic acid

In addition, thiophene, furanyl, pyrazine, pyrazole, pyridazine, thadiazole, and pyrrole groups function as isosteres for phenyl groups, and may be substituted for the phenyl group of the above bicyclic benzyl derivatives.

Representative derivatives of the present invention can be prepared according to the following illustrative synthetic schemes:

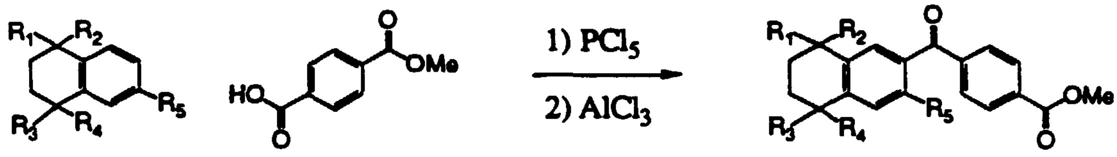


1 2 3

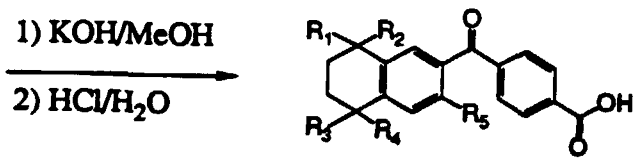


4

Compounds of structure 1 containing $R_5$ = lower alkyl are prepared in accordance with United States Patent No. 2,897,237. When $R_5$ = Halo, OH, amino, or thio, the products are prepared by standard Friedel-Crafts reaction conditions combining the appropriate substituted benzene with 2,5-dichloro-2,5-dimethyl hexane in the presence of aluminum trichloride.

Condensation of 1 with mono-methyl terephthalate 2 was carried out by addition of $PCl_5$ to 1 and 2 in $CH_2Cl_2$ followed by addition of $AlCl_3$ at room temperature.

The resulting methyl esters 3 are hydrolyzed to the carboxylic acid 4 by refluxing in aqueous KOH-MeOH followed by acidification.

4

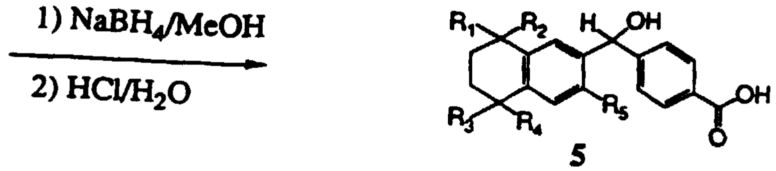


5

3

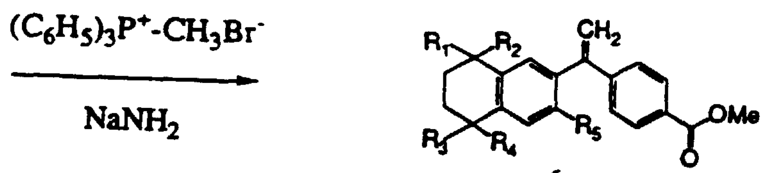


6

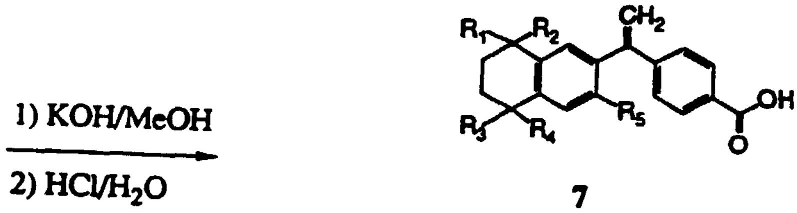


7

Treatment of ketone 4 with $NaBH_4$ afforded alcohol 5.

Treatment of the methyl ester 3 with methyltriphosphonium bromide-sodium amide in THF afforded the methano compound 6.

The carboxylic acid 7 was formed by adding KOH to methano compound 6 in MeOH, followed by acidification.

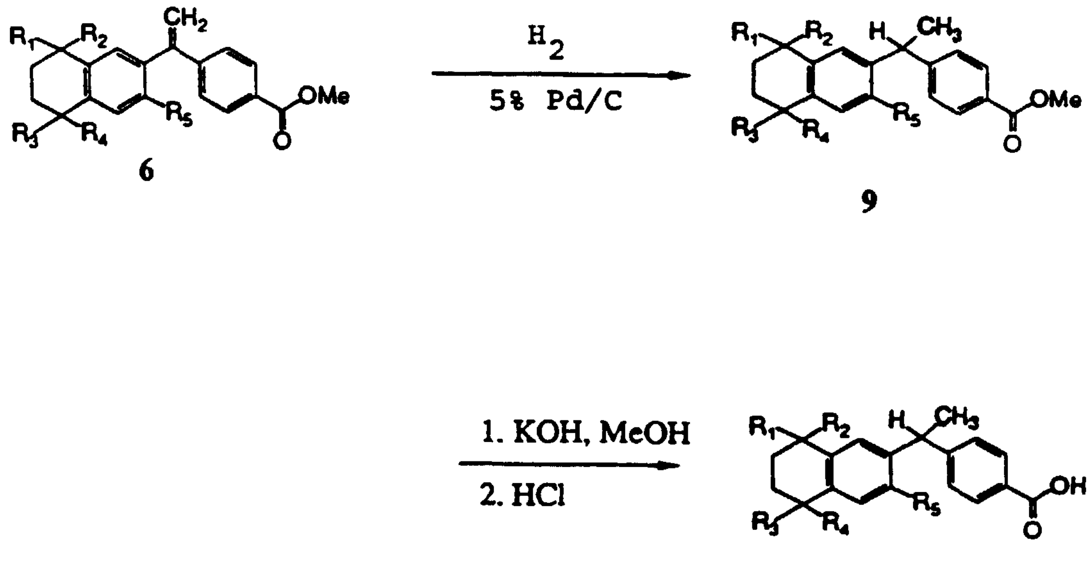


6 9 10

Treatment of the methyl ester 6 with hydrogen gas and 5% palladium over carbon in ethyl acetate yields the hydrogenated compound 9.

Treatment of compound 9 with aqueous KOH in refluxing MeOH, followed by acidification, yields the carboxylic acid compound 10.

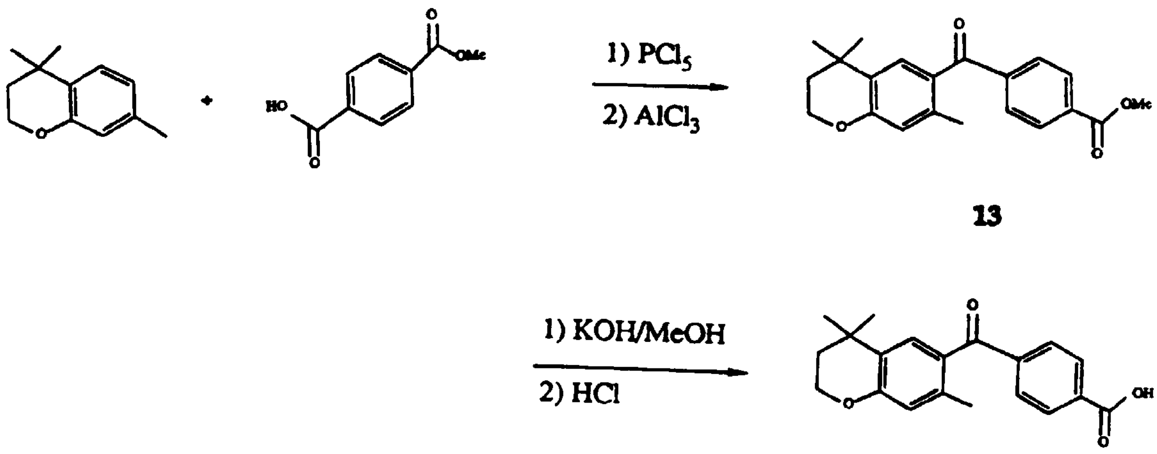


13 14

4,4-Dimethylchroman and 4,4-dimethyl-7-alkylchroman compounds of type 13 and 14 as well as 4,4-dimethylthiochroman, 4,4-dimethyl-7-alkylthiochroman, 4,4-dimethyl-1,2,3,4-tetrahydroquinoline, and 4,4-dimethyl-7-alkyl-1,2,3,4-tetrahydroquinoline analogs were synthesized by similar methods as compound 3, *i.e.*, Friedel-Crafts conditions combining the appropriate dimethylchroman, dimethylthiochroman or dimethyltetrahydroquinoline with mono-methyl terephthalate acid chloride in the presence of $AlCl_3$ or $SnCl_4$, followed by base hydrolysis and acidification to give the carboxylic acid. For the synthesis of the tetrahydroquinoline analogs, it was necessary to acylate the amine before Friedel-Crafts coupling with mono-methyl terephthalate acid chloride. For the synthesis of the appropriate dimethylchromans, dimethylthiochromans and tetrahydroquinolines, see U.S. Patent Nos. 5,053,523 and 5,023,341 and European Patent Publication No. 0284288.

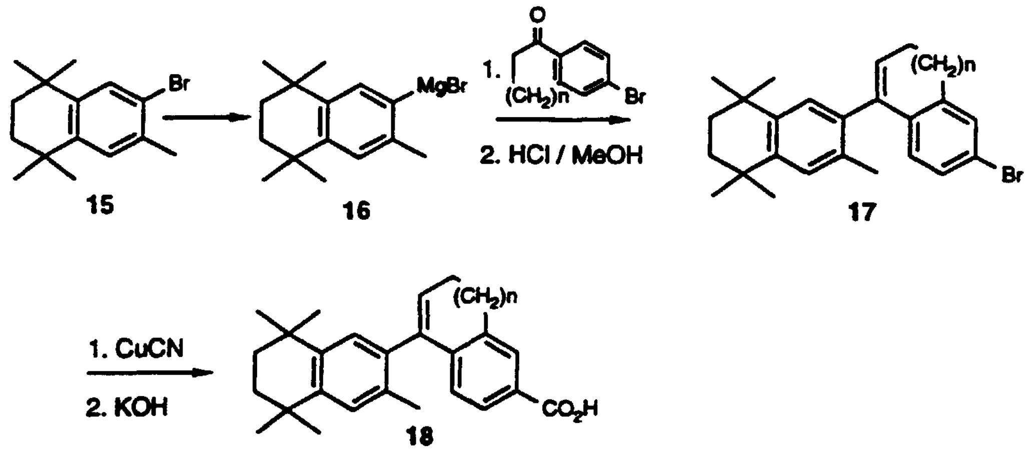


Compounds of the type 18 were synthesized by nucleophillic addition of the Grignard reagent 16 to bromotetralone, bromoindane, or other bicyclic ketone derivitive. Treatment of the resulting alcohol with methanolic HCl gave the intermediate 17. Displacement of the bromine with CuCN in quinoline gave the nitrile which was then hydrolyzed to the acid 18 in refluxing KOH. Bromine compound 15 was synthesized from 2,5-dichloro-2,5-dimethylhexane and 2-bromotoluene with a catalytic amount of $AlCl_3$.

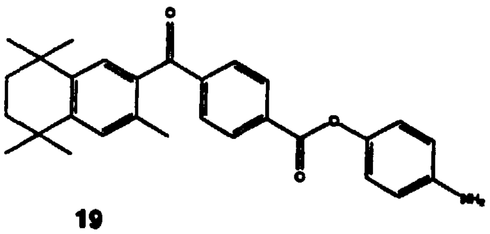

19

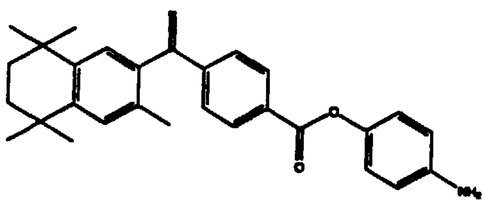

20

Treatment of compounds 3-methyl-TTNCB and 3-methyl-TTNEB with DCC, *p*-amino phenol, and DMAP resulted in the aminoesters 19 and 20.

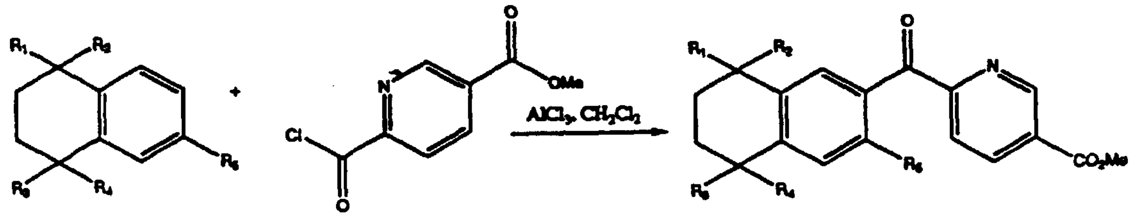


1 24 25

25

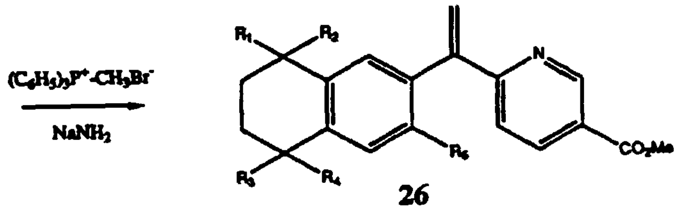


26

26

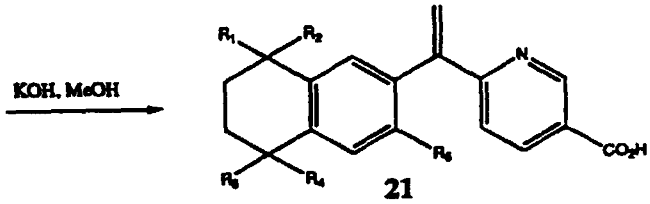


21

26

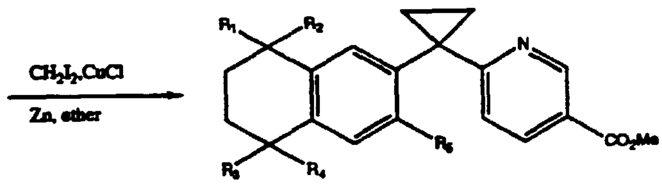


27

27

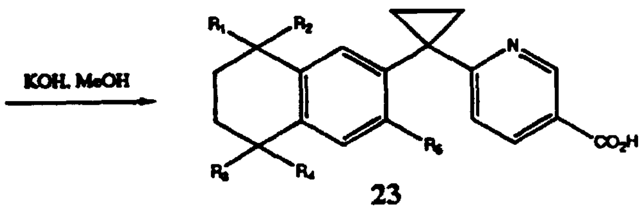


23

7

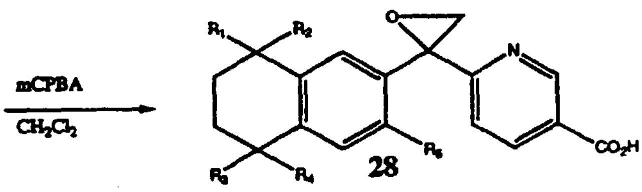


28

It may also be noted that pyridinal derivatives not within the present claims (compounds 21, 23, 26, and 27) may be prepared according to the illustrative synthetic schemes shown above. The synthesis of compound 21 is similar to that previously described for compound 7. Pentamethyl tetrahydronaphthalene 1, pyridinal acid chloride 24, and $AlCl_3$, are stirred in $CH_2Cl_2$ to give the ketone 25. Treatment of the ketone 25 with methyl triphosphonium bromide-sodium amide in THF afforded the ethenyl compound 26. Hydrolysis of 26 (KOH,MeOH) followed by acidification gave the acid 21. The cyclopropyl analog 23 was synthesized by treatment of the ethenyl compound 26 with $CH_2I_2$, zinc dust,

CuCl in refluxing ether (Simmons-Smith reaction). Hydrolysis of the resulting cyclopropyl ester 27 was achieved with methanolic KOH followed by acidification to give compound 23. When $R_1$-$R_5$ are methyl, for example, compound 62 (TPNCP) is obtained, as shown in Example 33 below. Other cyclopropyl derivatives such as TPNCB (compound 48) may be likewise prepared by the same method as described for analog 23: olefin 6 is treated with the Simmons-Smith reagent described above, followed by hydrolysis with methanolic-KOH and acidification (HCl) to give the desired cyclopropyl derivative. Epoxy derivatives such as TPNEB (compound 47) may be synthesized by treatment of compound 7 with m-chloroperbenzoic acid at room temperature in $CH_2Cl_2$ for several hours.

Illustrative examples for the preparation of some of the compounds according to this invention are as follows:

Example 1

Preparation of compound 3 where $R_1$, $R_2$, $R_3$, $R_4$ and $R_5$ are methyl, R' and R" are oxo, and X=COOMe:

To 7 gm (34.7 mmol) of 1,1,4,4,6-pentamethyl-1,2,3,4-tetrahydronaphthalene and 6 gm (33.3 mmol) of mono-methyl teraphthalate in 200 mL of $CH_2Cl_2$ was added 8 g (38.8 mmol) of $PCl_5$. The reaction boiled vigorously and turned clear within 10 min. After stirring for an additional 1 h, 6 g (43.5 mmol) of $AlCl_3$ was added in 1 g portions over 15 min. and the reaction was allowed to stir overnight. The mixture was poured into 300 mL of 20% aqueous HCl and extracted with 5% EtOAc-hexanes, dried (MgSO4), concentrated, and crystallized from MeOH to give ca. 6 gm (16.5 mmol) of methyl ester 3.

$^1$HNMR ($CD_3OCD_3$) δ 1.20 (s, 2($CH_3$)), 1.35 (s, 2($CH_3$)), 1.75 (s, 2($CH_2$)), 2.31 (s, $CH_3$), 3.93 (s, $COOCH_3$), 7.21 (s, Ar-CH), 7.23 (s, Ar-CH), 7.85 (d, J=8 Hz, Ar-2(CH)), 8.18 (d, J=8 Hz, Ar-2(CH)).

Example 2

Preparation of compound 4 where $R_1$, $R_2$, $R_3$, $R_4$ and $R_5$ are methyl, R' and R" are oxo, and X = COOH (3-methyl-TTNCB):

To 6 gm (16.5 mmol) of methyl ester 3 suspended in 100 mL of MeOH was added 50 mL of 5N aqueous KOH. The mixture was heated under reflux for 1 h, cooled, acidified (20% aqueous HCl) and the organics extracted with EtOAc. After drying (MgSO4), the product was concentrated and precipitated from 1:4 EtOAc-hexanes to give ca. 5 g (14.3 mmol) of acid 4.

$^1$HNMR ($CD_3OCD_3$) δ 1.20 (s, 2($CH_3$)), 1.35 (s, 2($CH_3$)), 1.75 (s, 2($CH_2$)), 2.31 (s, $CH_3$), 7.21 (s, Ar-CH), 7.23 (s, Ar-CH), 7.91 (d, J=8 Hz, Ar-2(CH)), 8.21 (d, J=8 Hz, Ar-2(CH)).

Example 3

Preparation of compound 5 where $R_1$, $R_2$, $R_3$, $R_4$ and $R_5$ are methyl, R' = H and R" = OH, and X = COOH (3-methyl-TTNHMB):

To a 1:1 THF-MeOH solution containing 1 g (2.86 mmol) of ketone 4 was added 100 mg of $NaBH_4$. The mixture was heated to 50°C for 10 min., cooled, acidified (20% aqueous HCl), and the organics extracted (EtOAc). After drying ($MgSO_4$), the product was concentrated and precipitated from 1:3 EtOAc-hexanes to give 550 mg (1.56 mmol) of the alcohol 5.

$^1$HNMR ($CD_3OCD_3$) δ 1.20 (s, $CH_3$)), 1.22 (s,($CH_3$)), 1.22 (s, 2($CH_3$)), 1.65 (s, 2($CH_2$)), 2.21 (s, $CH_3$), 6.00 (s, -CHOH-), 7.09 (s, Ar-CH), 7.41 (s, Ar-CH), 7.53 (d, J=8 Hz, Ar-2(CH)), 8.01 (d, J=8 Hz, Ar-2(CH)).

Example 4

Preparation of compound 6 where $R_1$, $R_2$, $R_3$, $R_4$ and $R_5$ are methyl, R' and R" are methano, and X = COOMe:

To 1 gm of methyl ester 3 (2.7 mmol) in 25 mL of dry THF was added 1.2 g (3.08 mmol) of methyltriphosphonium bromide-sodium amide. The solution was stirred at RT for 3 h or until complete by TLC (20% EtOAc-hexanes). Water was added and the organics were extracted with EtOAc, dried ($MgSO_4$), concentrated and purified by $SiO_2$ chromatography (5% EtOAc-hexanes) followed by crystallization from MeOH to give 700 mg (1.93 mmol) of methano compound 6.

$^1$HNMR ($CD_3OCD_3$) δ 1.22 (s, 2($CH_3$)), 1.30 (s, 2($CH_3$)), 1.72 (s, 2($CH_2$)), 1.95 (s, $CH_3$), 3.85 (s, $COOCH_3$), 5.29 (s,=CH), 5.92 (s, =CH), 7.19 (s, Ar-CH), 7.20 (s, Ar-CH), 7.39 (d, J=8 Hz, Ar-2(CH)), 7.96 (d, J=8 Hz, Ar-2(CH)).

Example 5

Preparation of compound 7 where $R_1$, $R_2$, $R_3$, $R_4$ and $R_5$ are methyl, R' and R'' are methano, and X = COOH (3-methyl-TTNEB):

To 500 mg of methano compound 6 (1.38 mmol) in 20 mL of MeOH was added 5 mL of 5 N aqueous KOH and the suspension was refluxed for 1 h. After acidification (20% aqueous HCl) the organics were extracted (EtOAc), dried ($MgSO_4$), concentrated, and the solids recrystallized from EtOAc-hexanes 1:5 to give 350 mg (1.0 mmol) of the carboxylic acid 7.

$^1$HNMR ($CD_3OCD_3$), δ 1.22 (s, 2($CH_3$)), 1.30 (s, 2($CH_3$), 1.72 (s, 2($CH_2$)), 1.95 (s, $CH_3$), 5.22 (s,=CH), 5.89 (s,=CH), 7.19 (s, Ar-CH), 7.20 (s, Ar-CH), 7.39 (d, J=8 Hz, Ar-2(CH)), 7.96 (d, J = 8 Hz, Ar-2(CH)).

Example 6

Preparation of compound 37 where $R_1$,$R_2$,$R_3$,$R_4$ are methyl, $R_5$ is isopropyl, R' and R'' are oxo, and X=COOR (3-IPR-TTNCB):

The compound was prepared in a manner similar to that of compound 4 except that 6-isopropyl-1,1,4,4-tetramethyl-1,2,3,4-tetra-hydronaphthalene was substituted for 1,1,4,4,6-pentamethyl-1,2,3,4-tetrahydronaphthalene in examples 1 and 2. MP: 254 °C; $^1$H-NMR ($CDCl_3$) δ1.19 (d,J=7 Hz,$CH(CH_3)_2$), 1.21 (s,2($CH_3$), 1.33 (s,2($CH_3$), 1.70 (s,2($CH_2$)), 3.12 (q,J=7 Hz,$CH(CH_3)_2$), 7.14 (s,Ar-CH), 7.37 (s,Ar-CH), 7.92 (d,J=8 Hz, Ar-2(CH)), 8.18 (d,J=8 Hz, Ar-2(CH)).

Example 7

Preparation of compound 38 where $R_1$,$R_2$,$R_3$,$R_4$ are methyl, $R_5$ is chloro, R' and R'' are oxo, and X=COOH (3-chloro-TTNCB):

The compound was prepared in a manner similar to that of compound 4 except that 6-chloro-1,1,4,4-tetramethyl-1,2,3,4-tetrahydro-naphthalene was substituted for 1,1,4,4,6-pentamethyl-1,2,3,4-tetrahydronaphthalene in examples 1 and 2. MP: 254 °C; $^1$H-NMR ($CDCl_3$) δ1.26 (s,2($CH_3$)), 1.32 (s,2($CH_3$)), 1.72 (s,2($CH_2$)), 7.35 (s,Ar-CH), 7.36 (s,Ar-CH), 7.91 (d,J=8 Hz, Ar-2(CH)), 8.19 (d,J=8 Hz, Ar-2(CH)).

Example 8

Preparation of compound 39 where $R_1$,$R_2$,$R_3$,$R_4$ are methyl, $R_5$ is hydroxy, R' and R'' are oxo, and X = COOH (3-hydroxy-TTNCB):

The compound was prepared in a manner similar to that of compound 4 except that 6-hydroxy-1,1,4,4-tetramethyl-1,2,3,4-tetrahydro-naphthalene was substituted for 1,1,4,4,6-pentamethyl-1,2,3,4-tetrahydronaphthalene in examples 1 and 2. MP: 264 °C; $^1$H-NMR ($CDCl_3$) δ1.17 (s,2($CH_3$)), 1.31 (s,2($CH_3$)), 1.68 (s,2($CH_2$)), 7.02 (s,Ar-CH), 7.44 (s,Ar-CH), 7.77 (d,J=8 Hz, Ar-2(CH)), 8.27 (d,J=8 Hz, Ar-2(CH)), 11.50 (s,-OH).

Example 9

Preparation of compound 40 where $R_1$,$R_2$,$R_3$,$R_4$ are methyl, $R_5$ is ethyl, R' and R'' are oxo, and X=COOH (3-Et-TTNCB):

The compound was prepared in a manner similar to that of compound 4 except that 6-ethyl-1,1,4,4-tetramethyl-1,2,3,4-tetrahydro-naphthalene was substituted for 1,1,4,4,6-pentamethyl-1,2,3,4-tetrahydronaphthalene in examples 1 and 2. MP: 226°C; $^1$H-NMR ($CDCl_3$) δ1.16 (t,J=7.5 Hz,-$CH_2CH_3$), 1.19 (s,2($CH_3$)), 1.32 (s,2($CH_3$)), 1.69 (s,2($CH_2$)), 2.69(q,J=7.5 Hz, $CH_2CH_3$), 7.20 (s,Ar-CH), 7.25 (s,Ar-CH), 7.87 (brd,Ar-2(CH)), 8.20 (brd,Ar-2(CH)).

Example 10

Preparation of compound 41 where $R_1$,$R_2$,$R_3$,$R_4$ are methyl, $R_5$ is bromo, R' and R" are oxo, and X = COOH (3-bromo-TTNCB):

The compound was prepared in a manner similar to that of compound 4 except that 6-bromo-1,1,4,4-tetramethyl-1,2,3,4-tetrahydro-naphthalene was substituted for 1,1,4,4,6-pentamethyl-1,2,3,4-tetrahydronaphthalene in examples 1 and 2. MP: 275 °C; $^1$H-NMR ($CDCl_3$) δ1.25 (s,2($CH_3$), 1.32 (s,2($CH_3$)), 1.71 (s,2($CH_2$)), 7.30 (s,Ar-CH), 7.54 (s,Ar-CH), 7.90 (d,J=8 Hz, Ar-2(CH)), 8.18 (d,J=8 Hz,Ar-2(CH)).

Example 11

Preparation of compound 42 where $R_1$,$R_2$,$R_3$,$R_4$ are methyl, $R_5$ is isopropyl, R' and R" are methano, and X = COOH (3-IPR-TTNEB):

The compound was prepared in a manner similar to that of compound 7 except that 6-isopropyl-1,1,4,4-tetramethyl-1,2,3,4-tetrahydro-naphthalene was substituted for 1,1,4,4,6-pentamethyl-1,2,3,4-tetrahydronaphthalene in examples 1, 2, 4, and 5. MP: 252 °C; $^1$H-NMR ($CDCl_3$) δ1.05 (d,J=7 Hz,CH($CH_3$)$_2$), 1.27 (s,2($CH_3$)), 1.32 (s,2($CH_3$)), 1.70 (s,2($CH_2$)), 2.73(q,J=7 Hz,CH($CH_3$)$_2$), 5.32 (s,=CH), 5.87 (s,=CH) 7.06 (s,Ar-CH), 7.23 (s,Ar-CH), 7.40 (d,J=8 Hz,Ar-2(CH)), 8.040 (d,J=8 Hz,Ar-2(CH)).

Example 12

Preparation of compound 43 where $R_1$,$R_2$,$R_3$,$R_4$ are methyl, $R_5$ is chloro, R' and R" are methano, and X=COOH (3-chloro-TTNEB):

The compound was prepared in a manner similar to that of compound 7 except that 6-chloro-1,1,4,4-tetramethyl-1,2,3,4-tetrahydro-naphthalene was substituted for 1,1,4,4,6-pentamethyl-1,2,3,4-tetrahydronaphthalene in examples 1, 2, 4, and 5. MP: 233 °C; $^1$H-NMR ($CDCl_3$) δ1.28 (s,2($CH_3$)), 1.31 (s,2($CH_3$)), 1.71 (s,2($CH_2$)), 5.42 (s,=CH), 5.89 (s,=CH), 7.23 (s,Ar-CH), 7.28 (s,Ar-CH), 7.37 (d,J=8 Hz,Ar-2(CH)), 8.03 (d,J=8 Hz,Ar-2(CH)).

Example 13

Preparation of compound 44 where $R_1$,$R_2$,$R_3$,$R_4$ are methyl, $R_5$ is hydroxy, R' and R" are methano, and X = COOH (3-hydroxy-TTNEB):

The compound was prepared in a manner similar to that of compound 7 except that 6-hydroxy-1,1,4,4-tetramethyl-1,2,3,4-tetrahydro-naphthalene was substituted for 1,1,4,4,6-pentamethyl-1,2,3,4-tetrahydronaphthalene in examples 1, 2, 4, and 5. MP: 216 °C; $^1$H-NMR ($CDCl_3$) δ1.21 (s,2($CH_3$), 1.30 (s,2($CH_3$)), 1.68 (s,2($CH_2$)), 5.54 (s,=CH), 5.94 (s,=CH), 6.86 (s,Ar-CH), 7.00 (s,Ar-CH), 7.48 (d,J=8.4 Hz, Ar-2(CH)), 8.07 (d,J=8.4 Hz, Ar-2(CH)).

Example 14

Preparation of compound 45 where $R_1$,$R_2$,$R_3$,$R_4$ are methyl, $R_5$ is ethyl, R' and R" are methano, and X = COOH (3-Et-TTNEB):

The compound was prepared in a manner similar to that of compound 7 except that 6-ethyl-1,1,4,4-tetramethyl-1,2,3,4-tetrahydro-naphthalene was substituted for 1,1,4,4,6-pentamethyl-1,2,3,4-tetrahydronaphthalene in examples 1, 2, 4, and 5. MP: 236 °C; $^1$H-NMR ($CDCl_3$) δ0.99 (t,J=7.6 Hz,-$CH_2CH_3$), 1.27 (s,2($CH_3$)), 1.31 (s,2($CH_3$)), 1.70 (s,2($CH_2$)), 2.29(q,J=7.6 Hz,-$CH_2CH_3$), 5.34 (s,=CH), 5.83 (s,=CH), 7.08 (s,Ar-CH), 7.12 (s,Ar-CH), 7.38 (d,J=8 Hz, Ar-2(CH)), 8.00 (d,J=8 Hz, Ar-2(CH)).

Example 15

Preparation of compound 46 where $R_1$,$R_2$,$R_3$,$R_4$ are methyl, $R_5$ is bromo, R' and R" are methano, and X = COOH (3-bromo-TTNEB):

The compound was prepared in a manner similar to that of compound 7 except that 6-bromo-1,1,4,4-tetramethyl-1,2,3,4-tetrahydro-naphthalene was substituted for 1,1,4,4,6-pentamethyl-1,2,3,4-tetrahydronaphthalene in examples 1, 2, 4, and 5. MP: 235 °C; $^1$H-NMR ($CDCl_3$) δ1.27 (s,2($CH_3$)), 1.31 (s,2($CH_3$)), 1.71 (s,$CH_3$), 5.40 (s,=CH), 5.90 (s,=CH), 7.26 (s,Ar-CH), 7.36 (s,Ar-CH), 7.43 (d,J=8 Hz,Ar-2(CH)), 8.04 (d,J=8 Hz,Ar-2(CH)).

Example 16

Preparation of compound 47 where $R_1$,$R_2$,$R_3$,$R_4$,$R_5$ are methyl, R' and R" taken together are $CH_2$-O (epoxide), and X = COOH (TPNEB):

The compound was prepared from compound 6 where $R_1$, $R_2$, $R_3$, $R_4$, $R_5$ are methyl. To 1 g (2.76 mmol) of olefin 6 in 5 mL of $CH_2Cl_2$ was added 600 mg (3.46 mmol) of mCPBA and the reaction was stirred at room temperature for 2 h. Water was added followed by extraction of the organics with ether. The ether layer was washed with water, 1N $Na_2CO_3$, brine and dried ($MgSO_4$), filtered and concentrated. Crystallization from MeOH gave the desired epoxide-methyl ester. The methyl ester was hydrolized in refluxing methanolic KOH followed by acidification (1N HCl) to give the crude epoxide-acid 47 which was purified by crystallization from EtOAc-hex to give 600 mg (1.64 mmol) of a white powder (59% yield). MP: 168 °C; $^1$H-NMR ($CDCl_3$) δ1.26 (s,$CH_3$), 1.27 (s,$CH_3$), 1.30 (s,$CH_3$), 1.31 (s,$CH_3$), 1.69 (s,(2$CH_2$)), 2.14 (s,$CH_3$), 3.15 (d,J=5.6 Hz,CH-O), 3.41 (d,J=5.6 Hz,CH-O), 7.09 (s,Ar-CH), 7.28 (d,J=8.3 Hz, Ar-2(CH)), 7.32 (s,Ar-CH), 8.01 (d,J=8.3 Hz,Ar-2(CH)).

Example 17

Preparation of compound 48 where $R_1$,$R_2$,$R_3$,$R_4$,$R_5$ are methyl, R' and R" taken together are $CH_2$-$CH_2$(cyclopropyl), and X = COOH (TPNCB):

The compound was prepared from compound 6 where $R_1$, $R_2$, $R_3$, $R_4$, $R_5$ are methyl. To a dry 100 mL three necked round bottom flask fitted with a reflux condensor, dropping funnel, and magnetic stir bar was added 722 mg (11.65 mmol) of zinc dust, 109 mg (1.105 mmol) of cuprous chloride (CuCl), 7.5 mL of dry THF, and 1.48 g (5.52 mmol) of diiodomethane. To the addition funnel is added 1g (2.76 mmol) of compound 6 in 5 mL of dry THF. The flask is heated to 80 °C, followed by dropwise addition of 6. After the addition of 6 was complete, the reaction was allowed to reflux for 30 h or until completion, followed by dilution with 50 mL of ether and 20 mL of saturated aqueous ammonium chloride solution. The organic layer was washed with 10% NaOH (3 x 20 mL), brine and dried over anhydrous $MgSO_4$. The product was concentrated and purified by preparative TLC (2% EtOAc-hexane) to give 220 mg (0.59 mmol) of the methyl ester of 48. Hydrolysis of the methyl ester with refluxing methanolic KOH, followed by acidification (1N HCl), gave 150 mg (0.41 mmol) of the desired compound 48 after crystallization from EtOAc-hexane (15% yield). MP: 244 °C; $^1$H-NMR ($CDCl_3$) δ1.28 (s,4 ($CH_3$)), 1.39 Hz,Ar-2(CH)), 7.06 (s,Ar-CH), 7.29 (s,Ar-CH), 7.91 (d,J=8.4 Hz,Ar-2(CH)).

Example 18

Preparation of compound 49 where $R_1$,$R_2$,$R_3$,$R_4$,$R_5$ are methyl, R' = H and R" = $CH_3$, and X = COOH (PTNEB):

The compound was prepared from compound 7 where $R_1$, $R_2$, $R_3$, $R_4$, $R_5$ are methyl. To 1g (2.87 mmol) of compound 7 in 25 mL of EtOAc was added 10 mg of 10% Pd/C. The mixture was degassed under vacuum followed by addition of $H_2$, and allowed to stir under $H_2$ for 2 h. The reaction was filtered through celite and the product crystallized from EtOAc-hexane to give 750 mg (2.14 mmol) of the desired product 49 (75% yield). MP: 208 °C; $^1$H-NMR ($CDCl_3$) δ1.24 (s,$CH_3$), 1.25 (s,$CH_3$), 1.26 (s,$CH_3$), 1.29 (s,$CH_3$), 1.61 (d,J=7.2 Hz,$CH_3$), 1.67 (s,2($CH_2$)), 2.12 (s,$CH_3$), 4.30(q,J=7.2 Hz, CH), 7.02 (s,Ar-CH), 7.20 (s,Ar-CH), 7.24 (d,J=8.4 Hz, Ar-2(CH)), 7.99 (d,J=8.4 Hz,Ar-2(CH)).

Example 19

Preparation of compound 50 where $R_1$,$R_2$,$R_3$,$R_4$,$R_5$ are methyl, R' and R" = methylidene cyclopentane, and X = COOH (PTNCB):

The compound was prepared from compound 4 and where $R_1$, $R_2$, $R_3$, $R_4$, $R_5$ are methyl. To 1g (2.87 mmol) of 4 in 25 nit of TRF at 0 °C was added 8.6 mL of a 1M cyclopentenyl magnesium chloride solution (8.6 mmol). After stirring for 30 m, water was added and acidified with 5 N HCl. The acidified mixture was heated for 5 m, cooled, and the organic product extracted with EtOAc. The EtOAc layer was washed with water and brine, dried ($MgSO_4$), filtered, and concentrated to give the crude product. Crystallization from EtOAc-hexane gave 340 mg (0.85 mmol) of 50 as a white powder (30% yield). MP: 201 °C; $^1$H-NMR ($CDCl_3$) δ1.27 (s,4($CH_3$)), 1.64 (br t,$CH_2$), 1.68 (s,2($CH_2$)), 1.70 (br t,$CH_2$),

1.97 (s,$CH_3$), 2.15 (br t,$CH_2$), 2.56 (br t,$CH_2$), 7.04 (s,Ar-CH), 7.05 (s,Ar-CH), 7.29 (d,J=8 Hz,Ar-2(CH)), 7.97 (d,J=8 Hz,Ar-2(CH)).

Example 20

Preparation of compound 51 where $R_1$,$R_2$,$R_3$,$R_4$,$R_5$ are methyl, R' and R'' = isopropylidene, and X - COOH (PTNIB):

The compound was prepared from compound 4 and where $R_1$, $R_2$, $R_3$, $R_4$, $R_5$ are methyl. To 1g (2.87 mmol) of 4 in 25 mL of THF at 0°C was added 8.6 mL of a 1M isopropyl magnesium chloride solution (8.6 mmol). After stirring for 30 m, water was added and acidified with 5 N HCl. The acidified mixture was heated for 5 m, cooled, and the organic product extracted with EtOAc. The EtOAc layer was washed with water and brine, dried ($MgSO_4$), filtered, and concentrated to give the crude isopropylidene product. Crystallization from EtOAc-hexane gave 550 mg (1.46 mmol) of 51 as a white powder (51% yield). MP: 297°C; $^1$H-NMR ($CDCl_3$) δ1.25 (br s,4($CH_3$)), 1.64 (s,=$CCH_3$), 1.66 (s,=$CCH_3$) 1.87 (s,2($CH_2$)), 1.96 (s,$CH_3$), 7.00 (s,Ar-CH), 7.03 (s,Ar-CH), 7.25 (d,J=8 Hz,Ar-2(CH)), 7.97 (d,J=8 Hz,Ar-2(CH)).

Example 21

Preparation of compound 54 where $R_1$,$R_2$,$R_3$,$R_4$,$R_5$ are methyl, R' and R'' = oxo, and X = tetrazole (3-methyl-TTNCBT):

To 500 mg (1.51 mmol) of 4-[(3,5,5,8,8-pentamethyl-5,6,7,8-tetrahydro-2-naphthyl)carbonyl]benzonitrile (synthesized by $AlCl_3$ catalyzed condensation of 1,1,4,4,6-pentamethyl-1,2,3,4-tetra hydronaphthalene with 4-cyanobenzoic acid chloride in $CH_2Cl_2$) in toluene was added 342 mg (1.66 mmol) of trimethyl tin azide. The mixture was refluxed for 23 h and cooled to give 537 mg (1.44 mmol) of the desired tetrazole 54 as a white precipitate (96% yield). LRMS: 374.15; $^1$H-NMR ($CD_3SOCD_3$) δ1.19 (s,2($CH_3$)), 1.32 (s,2($CH_3$)), 1.70 (s,2($CH_2$)), 2.25 (s,$CH_3$), 3.19 (s,N-H), 7.30 (s,Ar-CH), 7.32 (s,Ar-CH), 7.90 (d,J=8 Hz,Ar-2(CH)), 8.20 (d,J=8 Hz,Ar-2(CH)).

Example 22

Preparation of compound 55 where $R_1$,$R_2$,$R_3$,$R_4$,$R_5$ are methyl, R' and R'' = methano, and X = tetrazole (3-methyl-TTNEBT):

To 500 mg (1.52 mmol) of 4-[1-(3,5,5,8,8-pentamethyl-5,6,7,8-tetrahydro-2-naphthyl)ethenyl]benzonitrile (synthesized by $AlCl_3$ catalyzed condensation of 1,1,4,4,6-pentamethyl-1,2,3,4-tetrahydronaphthalene with 4-cyanobenzoic acid chloride in $CH_2Cl_2$ followed by treatment of the ketone with $CH_3PPh_3Br$-$NaNH_2$) in toluene was added 342 mg (1.67 mmol) of trimethyl tin azide. The mixture was refluxed for 23 h and cooled to give 535 mg (1.44 mmol) of the desired tetrazole 55 as a white precipitate (95% yield). LRMS: 372.25; $^1$H-NMR ($CD_3SOCD_3$) δ1.21 (s,2($CH_3$)), 1.24 (s,2($CH_3$)), 1.68 (s,2($CH_2$)), 1.92 (s,$CH_3$), 2.55 (s,N-H), 5.27 (=CH), 5.97 (s,=CH), 7.10 (s,Ar-CH), 7.18 (s,Ar-CH), 7.47 (d,J=8 Hz,Ar-2(CH)), 8.00 (d,J=8 Hz,Ar-2(CH)).

Example 23

Preparation of compound 63 where $R_1$,$R_2$,$R_3$,$R_4$,$R_5$ are methyl, R' and R'' are methano, X = $CONHR_9$, and $R_9$ = 4-hydroxyphenyl (3-methyl-TTNEHBP):

To 750 mg (10 mml) of DMF in 22 mL of anhydrous ether was added 1.3g (10 mmol) of oxalyl chloride. The reaction was stirred for 1 h, followed by removal of solvent to give a crude white solid (dimethylchloroformadinium chloride). To the dimethylchloro-formadinium chloride was added 2.87 g (8.24 mmol) of compound 7 in 12 mL of dry DMF. The reaction was stirred for 20 m at room temperature followed by cooling to 0°C. The cooled solution of the acid chloride of 7 was added dropwise to a cooled DMF (0°C) solution containing 3.62 g (33 mmol) of 4-aminophenol and 1.68 g (16.3 mmol) of triethyl amine. After stirring at 0°C for 30 m, the reaction was warmed to room temperature for 12 h. Aqueous 20% HCl was added and the resulting solid was filtered and washed with water, acetone, and EtOAc to give 600 mg (1.36 mmol) of the desired compound 63 (17% yield). $^1$H-NMR ($CDCl_3$) δ1.29 (s,2($CH_3$)), 1.31 (s,2($CH_3$)), 1.71 (s,($CH_2$)), 1.99 (s,$CH_3$), 5.31 (s,=CH), 5.80 (s,=CH), 6.85 (d,Ar-2(CH)), 7.09 (s,Ar-CH), 7.16 (s,Ar-CH), 7.40 (d,Ar-2(CH)), 7.48 (d,Ar-2(CH)), 8.40 (d,Ar-2(CH)).

Example 24

Preparation of compound 64 where $R_1$,$R_2$,$R_3$,$R_4$,$R_5$ are methyl, R' and R'' are methano, X = $CONHR_9$, and $R_9$ = 4-fluorophenyl (3-methyl-TTNEFBP):

The compound was prepared in a manner similar to that of compound 63 except that 4-fluoroaniline was substituted for 4-aminophenol. MP: 203°C; $^1$H-NMR ($CDCl_3$) δ1.28 (s,2($CH_3$)), 1.31 (s,2($CH_3$)), 1.70 (s,2($CH_2$)), 1.96 (s,$CH_3$), 5.33 (s,=CH), 5.81 (s,=CH), 7.05 (d,J=9 Hz),Ar-2(CH)), 7.09 (s,Ar-CH), 7.13 (s,Ar-CH), 7.39 (d,J=8.4 Hz,Ar-2(CH)), 7.59 (dd,J=5,9 Hz,Ar-2CH), 7.75 (brs NH), 7.78 (d,J=8.4 Hz,Ar-2(CH)).

Example 25

Preparation of compound 65 where $R_1$,$R_2$,$R_3$,$R_4$,$R_5$ are methyl, R' and R'' are methano, X = $CONHR_9$, and $R_9$ = 4-phenylcarboxylic acid (3-methyl-TTNECBP):

The compound was prepared in a manner similar to that of compound 63 except that methyl 4-aminophenyl carboxylate was substituted for 4-aminophenol. The resulting ester was hydrolyzed in methanolic KOH, followed by acidification (20% HCl) to give the desired compound 65. MP: 200 °C; $^1$H-NMR ($CDCl_3$) δ1.28 (s,2($CH_3$)), 1.31 (s,2($CH_3$)), 1.71 (s,2($CH_2$)), 1.97 (s,$CH_3$), 5.34 (s,=CH), 5.85 (s,=CH), 7.09 (s,Ar-CH), 7.14 (s,Ar-CH), 7.40 (d,J=8 Hz,Ar-CH), 7.80 (d,J=8 Hz,Ar-2(CH)), 7.87 (br s,Ar-2(CH)), 8.14 (br s,Ar-2(CH)).

Example 26

Preparation of compound 66 where $R_1$,$R_2$,$R_3$,$R_4$,$R_5$ are methyl, R' and R'' are oxo, X = $CONHR_9$, and $R_9$ = 3-hydroxyphenyl (3-methyl-m-TTNCHBP):

To 750 mg (10 mml) of DMF in 22 mL of anhydrous ether was added 1.3g (10 mmol) of oxalyl chloride. The reaction was stirred for 1 h, followed by removal of solvent to give a crude white solid (dimethylchloroformadinium chloride). To the dimethylchlorofor-madinium chloride was added 2.88 g (8.24 mmol) of compound 4 in 12 mL of dry DMF. The reaction was stirred for 20 m at room temperature, followed by cooling to 0°C. The cooled solution of the acid chloride of 7 was added dropwise to a cooled DMF (0°C) solution containing 3.62 g (33 mmol) of 4-aminophenol and 1.68 g (16.3 mmol) of triethyl amine. After stirring at 0°C for 30 m, the reaction was warmed to room temperature for 12 h. Aqueous 20% HCl was added and the resulting solid was filtered and washed with water, acetone, and EtOAc to give 750 mg (1.70 mmol) of the desired compound 66 (21% yield). MP: 182°C; $^1$H-NMR ($CDCl_3$) δ1.22 (s,2($CH_3$)), 1.32 (s,2($CH_3$)), 1.70 (s,2($CH_2$)), 2.37 (s,$CH_3$), 6.58 (m,Ar-2(CH)), 7.20 (d,J=8 Hz,Ar-CH), 7.22 (s,Ar-CH), 7.28 (s,Ar-Ch), 7.91 (d,J=8.3 Hz,Ar-2(CH)), 8.26 (d,J=8.3 Hz,Ar-2(CH)).

Example 27

Preparation of compound 67 where $R_1$,$R_2$,$R_3$,$R_4$,$R_5$ are methyl, R' and R'' are methano, X = $CONHR_9$, and $R_9$ = 3-hydroxyphenyl (3-methyl-m-TTNEHBP):

The compound was prepared in a manner similar to that of compound 63 except that 3-aminophenol was substituted for 4-aminophenol. MP: 136°C; $^1$H-NMR ($CDCl_3$) δ1.28 (s,2($CH_3$)), 1.31 (s,2($CH_3$)), 1.70 (s,2($CH_2$)), 1.97 (s,$CH_3$), 5.35 (s,=CH), 5.84 (s,=CH), 6.57 (m,Ar-2(CH)), 7.09 (s,Ar-CH), 7.14 (s,Ar-CH), 7.16 (m,Ar-CH), 7.39 (d,J=8.3 Hz,Ar-2(CH)), 8.09 (d,J=8.3 Hz,Ar-2(CH)).

Example 28

Preparation of compound 68 where $R_1$,$R_2$,$R_3$,$R_4$,$R_5$ are methyl, R' and R'' are methano, X = $CONHR_9$, and $R_9$ = 2-hydroxyphenyl (3-methyl-o-TTNCHBP):

The compound was prepared in a manner similar to that of compound 63 except that 2-aminophenol was substituted for 4-aminophenol. MP: 180°C; $^1$H-NMR ($CDCl_3$) δ1.28 (s,2($CH_3$)), 1.31 (s,2($CH_3$)), 1.71 (s,2($CH_2$)), 1.97 (s,$CH_3$), 5.35 (s,=CH), 5.84 (s,=CH), 6.9 (m,Ar-CH), 7.08-7.2 (m,Ar-CH), 7.09 (s,Ar-CH), 7.13 (s,Ar-CH), 7.42 (d,J=8.4 Hz,Ar-2(CH)), 7.83 (d,J=8.4 Hz,Ar-2(CH)), 8.03 (brs,Ar-CH), 8.64 (s,NH).

Example 29

Preparation of compound 69 where $R_1$,$R_2$,$R_3$,$R_4$,$R_5$ are methyl, R' and R'' are methano, X = $CONHR_9$, and $R_9$ = 3-phenylcarboxylic acid (3-methyl-m-TTNECBP):

The compound was prepared in a manner similar to that of compound 63 except that methyl-3-amino phenyl carboxylate was substituted for 4-aminophenol. The resulting ester was hydrolyzed in methanolic KOH followed by acidification (20% HCl) to give the desired compound 69. MP: 250°C; $^1$H-NMR ($CDCl_3$) δ1.28 (s,2($CH_3$)), 1.31 (s,2($CH_3$)), 1.71 (s,2($CH_2$)), 1.97 (s,$CH_3$), 5.34 (s,=CH), 5.85 (s,=CH), 7.09 (s,Ar-CH), 7.14 (s,Ar-CH), 7.40 (d,J=8 Hz, Ar-2(CH)), 7.55 (m,Ar-CH),7.76 (m,Ar-CH), 7.80 (d,J=8 Hz, Ar-2(CH)), 7.87 (s,Ar-CH), 8.14 (s,NH).

Example 30

Preparation of compound 70 where $R_1$,$R_2$,$R_3$,$R_4$,$R_5$ are methyl, R' and R'' are methano, n=0, and X = COOH:

The compound was prepared in a manner similar to that of compound 7 except that 1,1,3,3,5-pentamethylindane was substituted for 1,1,4,4,6-pentamethyl-1,2,3,4-tetrahydronaphthalene in examples 1, 2, 4, and 5. MP: 145°C; $^1$H-NMR ($CDCl_3$) δ1.05 (s,2($CH_3$)), 1.28 (s,$CH_3$), 1.31 (s,$CH_3$), 1.38 (s,$CH_2$), 1.98 (s,$CH_3$), 5.34 (s,CH), 5.84 (s,CH), 6.90 (s, Ar-CH), 6.92 (s, Ar-CH), 7.36 (d,J=8.4 Hz, Ar-2 (CH)), 8.00 (d,J=8.4 Hz,Ar-2(CH)).

Example 31

Preparation of compound 71 where $R_1$,$R_2$,$R_3$,$R_4$,$R_5$,$R_{14}$ are methyl, R' and R'' are methano, n=0, and X = COOH:

The compound was prepared in a manner similar to that of compound 7 except that 1,1,2,3,3,5-pentamethylindane was substituted for 1,1,4,4,6-pentamethyl-1,2,3,4-tetrahydronaphthalene in examples 1, 2, 4, and 5. MP: 217°C; $^1$H-NMR ($CDCl_3$) δ1.01 (d,J=7.3 Hz,$CH_3$), 1.08 (s,$CH_3$), 1.10 (s,$CH_3$), 1.27 (s,$CH_3$), 1.30 (s,$CH_3$), 1.88 (q,CH), 2.00 (s,$CH_3$), 5.35 (s,=CH), 5.85 (s,=CH), 6.95 (s,Ar-CH), 6.98 (s,Ar-CH), 7.38(d,J=8.3 Hz,Ar-2(CH)), 8.00 (d,J=8.3 Hz,Ar-2(CH)).

Example 32

Preparation of compound 72 where $R_1$,$R_2$,$R_3$,$R_4$,$R_5$, are methyl, R' and R'' are H, and X = COOH:

The compound was prepared in a manner similar to that of compound 4 (examples 1 and 2) except that methyl-4-(bromomethyl)benzoate was substituted for mono-methyl terephthalic acid chloride. MP: 237°C; $^1$H-NMR ($CDCl_3$) δ1.23 (s,2($CH_3$)), 1.27 (s,2($CH_3$)), 1.67 (s,2($CH_2$)), 2.16 (s,$CH_3$), 4.06 (s,$CH_2$), 7.01 (s,Ar-CH), 7.08 (s,Ar-CH), 7.25 (d,J=8 Hz,Ar-2(CH)), 8.01 (d,J=8 Hz,Ar-2(CH)).

Evaluation of Retinoid Receptor Subtype Selectivity

Representative synthetic retinoid compounds of the current invention were analyzed and found to exhibit subtype selectivity for retinoid receptors, and to be capable of modulating processes selectively mediated by retinoid X receptors, as discussed more fully below.

As employed herein, the phrase "processes selectively mediated by retinoid X receptors" refers to biological, physiological, endocrinological, and other bodily processes which are mediated by receptors or receptor combinations which are responsive to retinoid X receptor selective processes, *e.g.*, compounds which selectively activate one and/or multiple members of the RXR subfamily. Modulation of such processes can be accomplished *in vitro* or *in vivo. In vivo* modulation can be carried out in a wide range of subjects, such as, for example, humans, rodents, sheep, pigs, cows, and the like.

The receptors which are responsive to retinoid X receptor selective ligands include: retinoid X receptor-alpha, retinoid X receptor-beta, retinoid X receptor-gamma, and splicing variants encoded by the genes for such receptors, as well as various combinations thereof (*i.e.*, homodimers, homotrimers, heterodimers, heterotrimers, and the like). Also included are combinations of retinoid X receptors with other members of the steroid/thyroid super-family of receptors with which the retinoid X receptors may interact by forming heterodimers, heterotrimers, and the higher heteromultimers. For example, the retinoic acid receptor-alpha, -beta, or -gamma isoforms form a heterodimer with any of the retinoid X receptor isoforms, (*i.e.*, alpha, beta, or gamma, including any combination of the different receptor isoforms), and the various retinoid X receptors form a heterodimer with thyroid receptor and form a heterodimer with vitamin D receptor. Members of the retinoid X receptor subfamily form a heterodimer with certain "orphan receptors" including PPAR (Issemann and Green, *Nature*, 347:645-49 (1990)); HNF4 (Sladek *et al., Genes & Development* 4:2353-65 (1990)); the COUP family of receptors (*e.g.*, Miyajima *et al*., *Nucleic Acids Research* 16:11057-74 (1988), and Wang *et al., Nature*, 340:163-66 (1989)); COUP-like receptors and COUP homologs, such as those described by Mlodzik *et al*. (*Cell*, 60:211-24 (1990)) and Ladias *et al. (Science*, 251:5561-65 (1991)); the ultraspiracle receptor (*e.g.*, Oro *et al., Nature*, 347:298-301 (1990)); and the like.

As employed herein, the phrase "members of the steroid/thyroid superfamily of receptors" (also known as "nuclear receptors" or "intracellular receptors") refers to hormone binding proteins that operate as ligand-dependent transcription factors. Furthermore, this classification includes identified members of the steroid/thyroid superfamily of receptors for which specific ligands have not yet been identified (referred to hereinafter as "orphan receptors"). All members of the intracellular receptor superfamily have the intrinsic ability to bind to specific DNA sequences. Following binding, the transcriptional activity of a target gene (*i.e.*, a gene associated with the specific DNA sequence) is modulated as a function of the ligand bound to the receptor. Also, see Heyman *et al.*, *Cell*, 68:397-406 (1992), and copending U.S. Serial No. 809,980, filed December 18, 1991, whose entire disclosures are incorporated herein by reference.

The modulation of gene expression by the ligand retinoic acid and its receptors can be examined in a reconstituted system in cell culture. Such a system was used to evaluate the synthetic retinoid compounds of this invention for their interaction with the retinoid receptor subtypes RARα, RARβ, RARγ, RXRα, RXRβ, and RXRγ.

The system for reconstituting ligand-dependent transcriptional control, which was developed by Evans *et al., Science*, 240:889-95 (1988), has been termed a "co-transfection" or *"cis-trans"* assay. This assay is described in further detail in U.S. Patent Nos. 4,981,784 and 5,071,773, which are incorporated herein by reference. Also see Heyman *et al., Cell,* 68:397-406 (1992). The co-transfection assay provides a mechanism to evaluate the ability of a compound to modulate the transcription response initiated by an intracellular receptor. The co-transfection assay is a functional, rapid assay that monitors hormone or ligand activity and is a good predictor of an *in vivo* system.

Briefly, the co-transfection assay involves the introduction of two plasmids by transient transfection into a retinoid receptor-negative mammalian cell background. The first plasmid contains a retinoid receptor cDNA and directs constitutive expression of the encoded receptor. The second plasmid contains a cDNA that encodes for a readily quantifiable protein, *e.g.*, firefly luciferase or chloramphenicol acetyl transferase (CAT), under control of a promoter containing a retinoid acid response element, which confers retinoid dependence on the transcription of the reporter. In this co-transfection assay, all retinoid receptors respond to all-*trans*-retinoic acid in a similar fashion. This assay can be used to accurately measure efficacy and potency of retinoic acid and synthetic retinoids as ligands that interact with the individual retinoid receptor subtypes.

Accordingly, synthetic retinoid compounds of the current invention were evaluated for their interaction with retinoid receptor subtypes using the co-transfection assay in which CV-1 cells were co-transfected with one of the retinoid receptor subtypes, a reporter construct, and an internal control to allow normalization of the response for transfection efficiency. The following example is illustrative.

Example 33

Retinoids: All-*trans*-retinoic acid (RA) and 13-*cis*-retinoic acid (13-*cis*-RA) were obtained from Sigma. 9-*cis*-retinoic acid (9-*cis*-RA) was synthesized as described in Heyman *et al.*, *Cell*, 68:397-406 (1992). Retinoid purity was established as greater than 99% by reverse phase high-performance liquid chromatography. Retinoids were dissolved in dimethylsulfoxide for use in the transcriptional activation assays.

Plasmids: The receptor expression vectors used in the co-transfection assay have been described previously (pRShRAR-α: Giguere *et al*. (1987); pRShRAR-β and pRShRAR-γ: Ishikawa *et al*. (1990); pRShRXR-α: Mangelsdorf *et al.*, (1990); pRSmRXR-β and pRSmRXR-γ: Mangelsdorf *et al., Genes & Devel.*, 6:329-44 (1992)). A basal reporter plasmid Δ-MTV-LUC (Hollenberg and Evans, *Cell*, 55:899-906 (1988)) containing two copies of the TRE-palindromic response element 5'-TCAGGTCATGACCTGA-3' (Umesono *et al., Nature,* 336:262-65 (1988)) was used in transfections for the RARs, and CRBPIIFKLUC, which contains an RXRE (retinoid X receptor response element (Mangelsdorf *et al*., *Cell*, 66:555-61 (1991)), was used in transfections for the RXRs.

Co-transfection Assay In CV-1 Cells: A monkey kidney cell line, CV-1, was used in the *cis-trans* assay. Cells were transfected with two plasmids. The trans-vector allowed efficient production of the retinoid receptor, in these cells, which do not normally express this receptor protein. The *cis*-vector contains an easily assayable gene product, in this case the firefly luciferase, coupled to a retinoid-responsive promoter, *i.e.*, an RARE or RXRE. Addition of retinoic acid or an appropriate synthetic retinoid results in the formation of a retinoid-RAR or - RXR complex that activates the expression of luciferase gene, causing light to be emitted from cell extracts. The level of luciferase activity is directly proportional to the effectiveness of the retinoid-receptor complex in activating gene expression. This sensitive and reproducible co-transfection approach permits the identification of retinoids that interact with the different receptor isoforms.

Cells were cultured in DMEM supplemented with 10% charcoal resin-stripped fetal bovine serum, and

experiments were conducted in 96-well plates. The plasmids were transiently transfected by the calcium phosphate method (Umesono and Evans, *Cell,* 57:1139-46 (1989) and Berger *et al., J.Steroid Biochem. Molec. Biol.*, 41:733-38 (1992)) by using 10 ng of a pRS (Rous sarcoma virus promoter) receptor-expression plasmid vector, 50 ng of the reporter luciferase (LUC) plasmid, 50 ng of pRSβ-GAL(β-galactosidase) as an internal control, and 90 ng of carrier plasmid, pGEM. Cells were transfected for 6 h and then washed to remove the precipitate. The cells were then incubated for 36 h with or without retinoid. After the transfection, all subsequent steps were performed on a Beckman Biomek Automated Workstation. Cell extracts were prepared, then assayed for luciferase and β-galactosidase activities, as described by Berger *et al*. (1992). All determinations were performed in triplicate in two independent experiments and were normalized for transfection efficiency by using β-galactosidase as the internal control. Retinoid activity was normalized relative to that of all-*trans*-retinoic acid and is expressed as potency (EC50), which is the concentration of retinoid required to produce 50% of the maximal observed response, and efficacy (%), which is the maximal response observed relative to that of all-*trans*-retinoic acid at $10^{-5}$M. The data obtained is the average of at least four independent experiments. Efficacy values less than 5% are not statistically different than the 0% background. Compounds with an efficacy of less than 20% at concentrations of $10^{-5}$ M are considered to be inactive. At higher concentrations of compound, such as $10^{-4}$ M, these compounds are generally toxic to cells and thus the maximal efficacy at $10^{-5}$ M is reported in the tables and figures contained herein.

The synthetic retinoid compound 3-methyl-TTNCB, as described above, was evaluated for its ability to regulate gene expression mediated by retinoid receptors. As shown in Figure 1, this compound is capable of activating members of the RXR subfamily, *i.e.*, RXRα, RXRβ, and RXRγ, but clearly has no significant activity for members of the RAR subfamily, *i.e.*, RARα, RARβ, and RARγ. Assays using all-*trans*-retinoic acid (Figure 2) and 9-*cis*-retinoic acid (Figure 3) were run for reference, and demonstrate that these retinoic acid isomers activate members of both the RAR and RXR subfamilies. Potency and efficacy were calculated for the 3-methyl-TTNCB compound, as summarized in the following table. For reference, the data for 9-*cis*-retinoic acid are also included.

TABLE 1

| | Potency (nM) | Efficacy |
|---|---|---|
| 3-Methyl-TTNCB | | |
| RXRα | 330 | 130% |
| RXRβ | 200 | 52% |
| RXRγ | 260 | 82% |
| RARα | >10,000 | <2% |
| RARβ | >10,000 | <4% |
| RARγ | >10,000 | <4% |
| 9-cis-retinoic acid | | |
| RXRα | 150 | 140% |
| RXRβ | 100 | 140% |
| RXRγ | 110 | 140% |
| RARα | 160 | 100% |
| RARβ | 5 | 82% |
| RARγ | 47 | 120% |

As shown by the data in Table 1, 3-methyl-TTNCB readily and at low concentrations activates RXRs. Further, 3-methyl-TTNCB is more potent an activator of RXRs than RARs, and preferentially activates RXRs in comparison to RARs, in that much higher concentrations of the compound are required to activate the RARs. In contrast, 9-*cis*-retinoic acid does not preferentially activate the RXRs, as also shown in Table 1. Rather, 9-*cis*-retinoic acid activates the RARβ and RARγ isoforms at lower concentrations and more readily than the RXRβ and RXRγ isoforms, and has substantially the same, within the accuracy of the measurement, activity for the RARα isoform in comparison to the RXRα isoform.

An extract reported to contain 9-*cis*-retinoic acid has previously been reported as at least 10-fold more potent in inducing RXRα than RARα (Heyman *et al*., *Cell*, 68:397,399 (January 24, 1992)). Presently available data

indicate that 9-*cis*-retinoic acid does not preferentially activate RXRs in comparison to RARs, as shown and discussed above. The compounds of this invention preferentially activate RXRs in comparison to RARs, and are preferably at least three times more potent as activators of RXRs than RARs, and more preferably at least five times more potent as activators of RXRs than RARs.

Potency and efficacy have also been calculated for the 3-methyl-TTNEB, 3-bromo-TTNEB, 3-methyl-TTNCHBP, and 3-methyl-TTNEHBP, compounds, as summarized below in Table 2.

TABLE 2

| | Potency (nM) | Efficacy |
|---|---|---|
| 3-Methyl-TTNEB | | |
| RXRα | 40 | 83% |
| RXRβ | 21 | 102% |
| RXRγ | 34 | 80% |
| RARα | >10,000 | 6% |
| RARβ | >10,000 | 17% |
| RARγ | >10,000 | 19% |
| 3-Bromo-TTNEB | | |
| RXRα | 64 | 88% |
| RXRβ | 54 | 49% |
| RXRγ | 52 | 71% |
| RARα | >10,000 | 3% |
| RARβ | >10,000 | 18% |
| RARγ | >10,000 | 15% |
| 3-Methyl-TTNCHBP | | |
| RXRα | 1100 | 113% |
| RXRβ | 1100 | 155% |
| RXRγ | 300 | 128% |
| RARα | >10,000 | <2% |
| RARβ | >10,000 | 7% |
| RARγ | >10,000 | 17% |
| 3-Methyl-TTNEHBP | | |
| RXRα | 140 | 125% |
| RXRβ | 71 | 121% |
| RXRγ | 48 | 163% |
| RARα | >10,000 | <2% |
| RARβ | 1,900 | 25% |
| RARγ | >10,000 | 10% |

As shown by the data in Table 2, 3-methyl-TTNEB, 3-bromo-TTNEB, 3-methyl-TTNCHBP, and 3-methyl-TTNEHBP, each readily and preferentially activate the RXRs, and are more potent as activators of RXRs than of RARs. The diminished activity of these compounds for the RARs in comparison to the RXRs is also shown for some of these compounds in Figures 4-7.

It can be expected that synthetic retinoid ligands, such as those exemplified in Tables 1 and 2 which preferentially affect some but not all of the retinoic acid receptor isoforms, can, in pharmacological preparations, provide pharmaceuticals with higher therapeutic indices and a better side effect profile than currently used retinoids. For example, the compounds of the present invention have been observed to be less irritating to the skin than standard retinoids.

The retinoid compounds of this invention are useful for the treatment of certain dermatological conditions such as keratinization disorders, *i.e.*, differentiation/proliferation. A standard assay to determine the activity of these compounds is the measurement of the enzymatic activity for transglutaminase; this is a measure of the antiproliferative action of retinoids. Retinoids have been shown to inhibit the pathway of differentiation, which is indicated by a decrease in several biochemical markers that are associated with the expression of squamous cell phenotype, such as transglutaminase. (Yuspa *et al., Cancer Research,* 43:5707-12 (1983)). As can be seen from Figure 8, the 3-methyl-TTNCB compound is capable of inhibiting transglutaminase activity and inhibits 50% of the enzyme activity at $1 \times 10^{-7}$ M.

The compounds of this invention also exhibit good comedolytic activity in the test on Rhino mice described by Kligman *et al. (J. Inves. Derm.*, 73:354-58 (1979)) and Mezick *et al.(J. of Inves. Derm.*, 83:110-13 (1984)). The test on Rhino mice has been a model for screening comedolytic agents. The activity of the 3-methyl-TTNCB retinoid compound, as well as 9-*cis* and all-*trans* retinoic acid is shown in Figure 9. A 0.1% solution of 3-methyl-TTNCB is capable of inhibiting the utriculi diameter by approximately 50%. It has also been observed that 3-methyl-TTNCB is less irritating to the skin of Rhino mice than 9-*cis*- or all-*trans*-retinoic acid.

The synthetic retinoids of the current invention have also been tested using radioligand displacement assays. RAR and RXR isoforms overexpressed in *E. coli* or baculovirus are capable of binding radiolabeled 9-*cis*-retinoic acid with binding parameters which are essentially similar to those receptors overexpressed in mammalian cells. By testing the abilities of various synthetic retinoids to compete with the radiolabeled retinoic acid for binding to various receptor isoforms, the relative dissociation constant for the receptor itself can be determined. This is an important supplementary analysis to the co-transfection assay since it can detect important discrepancies that may arise due to the various determinants of retinoid activity in the co-transfection assay. These determinants may include (1) activating or inactivating metabolic alterations in the test compounds, (2) binding to serum proteins which alter the free concentration of the test compound, (3) differences in cell permeation among test compounds, (4) intrinsic differences in the affinity of the test compounds for the receptor proteins, *i.e.*, in $K_d$, and (5) conformational changes produced in the receptor after binding of the test compound, reflected in the effects on reporter gene expression.

The 3-methyl-TTNCB compound is capable of displacing $^3$H-9-*cis*-retinoic acid bound to the RXRs, but is not capable of displacing radiolabeled ligand that is bound to the RARs. This indicates that the 3-methyl-TTNCB compound preferentially binds RXRs in comparison to RARs, a property which would be expected of a ligand selective for the RXRs.

It has been recognized that the co-transfection assay provides a functional assessment of the ligand being tested as either an agonist or antagonist of the specific genetic process sought to be affected. Ligands which do not significantly react with other intracellular receptors, as determined by the co-transfection assay, can be expected to result in fewer pharmacological side effects. Because the co-transfection assay is run in living cells, the evaluation of a ligand provides an early indicator of the potential toxicity of the candidate at concentrations where a therapeutic benefit would be expected.

Processes capable of being modulated by retinoid receptors, in accordance with the present invention, include *in vitro* cellular differentiation, the regulation of morphogenetic processes including limb morphogenesis, regulation of cellular retinol binding protein (CRBP), and the like. As readily recognized by those of skill in the art, the availability of ligands for the retinoid X receptor makes it possible, for the first time, to elucidate the processes controlled by members of the retinoid X receptor subfamily. In addition, it allows development of assays for the identification of antagonists for these receptors.

The processes capable of being modulated by retinoid receptors, in accordance with the present invention, further include the *in vivo* modulation of lipid metabolism; *in vivo* modulation of skin related processes (*e.g.*, acne, psoriasis, aging, wrinkling, and the like); *in vivo* modulation of programmed cell death (a poptosis); *in vivo* modulation of malignant cell development, such as occurs, for example, in acute promyelocytic leukemia, mammary cancer, prostate cancer, lung cancer, cancers of the aerodigestive pathway, skin cancer, bladder cancer, and sarcomas; *in vivo* modulation of premalignant lesions, such as occurs with oral leukoplakia and the like; *in vivo* modulation of auto-immune diseases such as rheumatoic arthritis; *in vivo* modulation of fatty acid metabolism; and the like. Such applications can be expected to allow the modulation of various biological processes with reduced occurrence of undesirable side effects such as teratogenic effects, skin irritation, mucosal dryness, lipid disturbances, and the like. *In vivo* applications can be employed with a wide range of subjects, such as, for example, humans, rodents, sheep, pigs, cows, and the like.

For example, regarding the *in vivo* modulation of lipid metabolism referred to above, apolipoprotein AI is a major protein component of plasma high density lipoprotein (HDL) cholesterol. Since the circulating level of HDL in humans has been shown to be inversely correlated to the risk of coronary vascular diseases, it can be expected that regulating synthesis of apolipoprotein AI can be utilized in the treatment of coronary vascular disease. It has been established that regulation of transcription of apolipoprotein AI is controlled by members of the intra-cellular receptor superfamily, and further that the apolipoprotein AI gene transcription start site A is a highly selective retinoid responsive

element (RXRE) that responds preferentially to RXRα. Rottman *et al., Mol. Cell. Biol.*, 11:3814-20 (1991). Therefore, ligands which selectively activate members of the RXR family of retinoic acid receptors may regulate apolipoprotein AI transcription. We have demonstrated in *in vivo* studies that ligands having selective activity for RXRs can be used to significantly raise plasma HDL levels, as demonstrated in the following example.

Example 34

Male Sprague-Dawley rats (160-200 gram) were obtained from Harland. Animals were fed standard laboratory diets (Harlan/Teklad) and kept in an environmentally controlled animal house with a light period lasting from 6 a.m. to 6 p.m. Animals were treated with drugs prepared as suspensions in olive oil.

To verify that RXR activation can modulate HDL cholesterol, an initial study was carried out that included dosing rats for 4 days with an RAR-selective compound, all-*trans* retinoic acid, the non-selective RAR/RXR agonist, 9-*cis*-retinoic acid, and either of two RXR-selective agents, 3-methyl-TTNCB or 3-methyl-TTNEB. Each drug was administered at a dose of 100 mg/kg, i.p. Positive control groups received olive oil as a vehicle. Twenty-four hours after the last treatment, rats were sacrificed by $CO_2$ inhalation, blood was collected from the inferior vena cava into a tube containing 0.1 ml of 0.15% EDTA and centrifuged at 1500 x g for 20 min. at 4°C. Plasma was separated and stored at 4°C for evaluation of plasma total cholesterol and high density lipoprotein cholesterol (HDL-cholesterol).

Plasma total cholesterol was measured enzymatically utilizing Boeringer Mannheim Diagnostics High Performance Cholesterol Methods with an ABBOTT VP Bichromatic Analyzer. HDL was measured after preparation of the HDL-containing fraction by heparin-manganese precipitation of plasma. HDL-cholesterol in this fraction was estimated as mentioned earlier. All HDL separations were checked for contamination by lipoproteins with agarose gel electrophoresis.

The results of this study are shown in Figure 10. As shown, rats receiving the RXR-selective compounds exhibited substantial and statistically significant increases in HDL levels, particularly when receiving 3-methyl-TTNEB. Because the RXR-selective ligand 3-methyl-TTNEB was the most efficacious, additional 4 day experiments were conducted with this agent at doses of 0.3, 1, 3, 6, 10, 30, 100, or 300 mg/kg i.p. in 0.5 ml olive oil or 1, 3, 10, 30, 100, 300 mg/kg p.o. in 0.5 ml olive oil for 4 days. An additional 30 day p.o. study was conducted with 10, 30, or 100 mg/kg 3-methyl-TTNEB to determine whether tolerance would develop to its pharmacological actions. For the rats receiving 3-methyl-TTNEB in various doses for four days, it was also observed that most of the HDL elevation was obtainable with relatively low doses (less than 5 mg/kg) of 3-methyl-TTNEB. The 30-day study with 3-methyl-TTNEB did not indicate development of tolerance to its pharmacological action.

Additional *in vitro* studies were also performed utilizing the co-transfection assay previously described within this application to demonstrate the effect of RXR-selective ligands on regulation of transcription of apolipoprotein-AI, as described in the following example.

Example 35:

This work focused on studying the transcriptional properties of the retinoid receptors RAR and RXR on a reporter molecule (*e.g.*, luciferase) under control of a basal promoter containing the RXR response element from the apolipoprotein AI gene ("A" site). Plasmid constructs coding for the various receptors were transfected into a human hepatocyte cell line (HepG-2) along with the reporter plasmid. Reporter plasmids contained multimers of the apolipoprotein-AI "A" site (-214 to -192 relative to transcription start site) shown to bind RXR. Widom *et al., Mol. Cell. Biol.* 12:3380-89 (1992); Ladias & Karathanasis, *Science* 251:561-65 (1991). After transfection, treatment, harvest, and assay, the data obtained was normalized to transfected beta-galactosidase activity so as to control for transfection efficiency. The results demonstrated activation in the system with the RXR-specific ligands 3-methyl-TTNCB and 3-methyl-TTNEB, demonstrating that the RXR specific ligands could regulate the transcriptional properties via the "A" site from the apolipoprotein AI gene. These compounds had no effect when RAR was used in the transfection, demonstrating receptor specificity. The transcriptional regulation by RXR was dependent on the presence of the hormone response element.

It has been surprisingly found that the administration of a compound containing a ligand which has specific activity for RXRs but essentially no activity for RARs, in combination with a ligand that has specific activity for RARS but not RXRs, provides a cellular response at extremely low dosages, dosages at which the ligands individually provide no significant response. Specifically, the concentration-related effect of an RXR-specific ligand and a RAR-specific ligand on proliferation of a myeloma cell line (RPMI 8226) was studied in *in vitro* studies using a thymidine incorporation assay. (L.M. Bradley, Selected Methods in Cellular Immunology, Ch. 10.1, pp. 235-38, Mishell & Shiigi (eds.), Freeman & Co., New York, 1980). This assay examines the incorporation of radiolabeled thymidine into DNA, and by determining the ability of a compound to inhibit thymidine incorporation into DNA, provides a measure of cell proliferation. Compounds which inhibit cell proliferation have well-known utility in the treatment of certain cancers.

As shown previously (Table 2), 3-methyl-TTNEB activates members of the RXR subfamily and has no significant activity for members of the RAR subfamily. Examination of the effects of 3-methyl-TTNEB on the proliferation of myeloma cells show a concentration dependent inhibition of thymidine incorporation. The $IC_{50}$ (the concentration of 3-methyl-TTNEB required to produce 50% inhibition of the maximal response) is $10^{-7}$ M, as shown in Figure 11. Concentrations less than $10^{-8}$ M provide essentially no effect on cell proliferation, as also shown in Figure 11.

It is well known that the compound TTNPB activates members of the RAR subfamily and has no significant activity for members of the RXR subfamily. The compound TTNPB is shown below, and its activity is shown in Table 3.

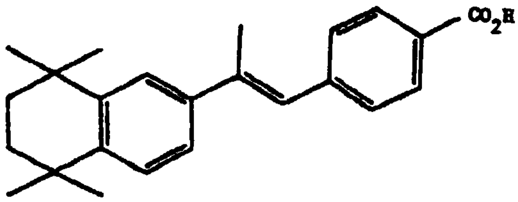

TTNPB

TABLE 3

| | Potency (nM) | Efficacy |
|---|---|---|
| TTNPB | | |
| RXRα | >10,000 | <5% |
| RXRβ | >10,000 | <5% |
| RXRγ | >10,000 | <5% |
| RARα | 52 | 30 |
| RARβ | 4 | 40 |
| RARγ | 0.4 | 50 |

The effect of TTNPB on cell proliferation is shown in Figure 11. The $IC_{50}$ value of TTNPB is about 5 x $10^{-11}$ M, and a concentration of less than $10^{-11}$ M produces essentially no effect on cell proliferation.

However, it has been found that when these two compounds (3-methyl-TTNEB and TTNPB) are present together, each at a concentration where the compound alone produces substantially no anti-proliferative effect, the combination of the two compounds effectively blocks cell proliferation. The combination of the two compounds appears to produce a greater than additive, or synergistic, effect.

For example, as shown in Figure 12, the presence of TTNPB at a concentration of $10^{-11}$ M produces a 9% inhibition on thymidine incorporation. However, combining it with 3-methyl-TTNEB at a concentration of $10^{-8}$ M (which results in no effect on cell proliferation) produces a greatly enhanced inhibitory effect of 49%. Likewise, it has also been found that the inhibitory effect of 3-methyl-TTNEB is greatly increased by the presence of TTNPB at a concentration which alone produces no effect.

Since it is well-known that toxic side effects of compounds such as TTNPB are concentration-dependent, the synergistic effect resulting from combining such RAR-specific compounds with RXR-specific compounds can be expected to permit lower dosages that are efficacious and to therefore reduce toxic side effects. For example, in cancer chemotherapy, use of two such compounds, in combination, at relatively low doses can be expected to produce the desired beneficial effect, while minimizing undesired side effects which result at higher doses of the compounds.

*In vitro* studies utilizing the co-transfection assay have also shown this same synergistic effect. For example, utilizing the co-transfection assay described previously and employing RAR-α and RXR-α and a reporter consisting of the ApoA1 response element "A" site in the context of TKLUC (Ladias & Karathanasis, *Science* 251:561-65 (1991), transfections were performed in HEPG2 cells. In this study, 100ng of the designated receptor were used and RSVCAT was used as a carrier to keep the amount of RSV promoter constant. All compounds were added at a final concentration of $10^{-7}$ M. The RXR specific compound, 3-methyl-TTNEB (Table 2, above), and the RAR specific compound, TTNPB (Table 3, above) were utilized. As shown below in Table 4, the relative normalized response observed utilizing the co-transfection assay also demonstrated a synergistic effect when a combination of the two compounds was utilized, compared to the response achieved utilizing the compounds individually.

TABLE 4

| Compound | Reporter Activity (Fold Induction) |
|---|---|
| 3-methyl-TTNEB | 5 |
| TTNPB | 32 |
| 3-methyl-TTNEB + TTNPB | 75 |

As will be discernable to those skilled in the art from the foregoing discussions, the biological response of an RAR selective compound at a given concentration can be synergistically enhanced by combining the compound with an RXR selective compound. Similarly, the biological response of an RXR selective compound can be enhanced by combining the compound with an RAR selective compound. Thus, it becomes possible to achieve a desirable biological response, using a combination of RAR and RXR selective compounds, at lower concentrations than would be the case using the compounds alone. Among the advantages provided by such combinations of RAR and RXR selective compounds are desirable therapeutic effects with fewer side effects. In addition, novel effects that are not obtainable with either agent alone may be achieved by combinations of RAR and RXR selective compounds.

It has been further demonstrated that RXR-specific compounds also synergistically enhance the response of other hormonal systems. Specifically, peroxisome proliferator-activated receptor (PPAR) is a member of the intracellular receptor super family that plays a role in the modulation of lipid homeostasis. PPAR has been shown to be activated by amphipathic carboxylates, such as clofibric acid, and these agents, called peroxisome proliferators, have been used in man as hypolipidemic agents. The addition of 9-cis-retinoic acid (a retinoid ligand which activates both RAR and RXR receptors) and clofibric acid to HepG2 cells transfected with RXRα and PPAR expression plasmids, results in the activation of receptor gene which was greater than the sum of the activation with each ligand separately. (Kliewer *et al., Nature* 358:771 (1992)). Similarly, when the above two receptors were co-transfected into HepG2 cells, the addition of both an RXR-specific ligand (3-methyl-TTNEB) and clofibric acid was found to produce a greater than additive response as determined by activation of a target reporter gene, as shown below in Table 5.

TABLE 5

| Compound | Normalized Response (%) |
|---|---|
| clofibric Acid | 100 |
| 3-methyl-TTNEB | 90 |
| clofibric acid + 3-methyl-TTNEB | 425 |

A similar synergistic effect was observed with RXR and RXR-specific ligands and the Vitamin D receptor (VDR) and its cognate ligands. When RXRβ and VD receptors were co-transfected into CV-1 cells containing a hormone response element, the addition of RXR selective 3-methyl-TTNCB and 1,25-dihydroxy-vitamin D (1,25-D) produced a greater than additive response than was observed for each of the individual ligands, as shown below in Table 6.

TABLE 6

| Compound | Normalized Response (%) |
|---|---|
| 1,25-D | 100 |
| 3-Methyl-TTNCB | 13 |
| 1,25-D + 3-methyl-TTNCB | 190 |

As shown, the above results indicate that each pair of receptors (RXRα/PPAR and RXRβ/VDR, respectively), in the presence of ligands known to specifically activate their respective receptors, are capable of producing a synergistic response. The results indicate that the response of a single agent can be enhanced by the combination of the two agents, or that comparable biological or therapeutic responses can be achieved by use of lower doses of such agents in combination.

The observation that RXR-specific ligands are able to act synergistically with RAR ligands, PPAR ligands, and Vitamin D ligands indicates that RXR-specific ligands have usefulness not only as single therapeutic agents but also in combination therapy to obtain enhanced biological or therapeutic response by the addition of the RXR-specific ligand. Such combination therapy also may provide an added benefit of decreasing the side effects associated with the primary agent by employing lower doses of that agent. For example, use of Vitamin D or a related Vitamin D receptor ligand in conjunction with an RXR selective compound for the treatment of a variety of disorders including skin diseases (acne, psoriasis), hyperproliferative disorders (benign and malignant cancers) and disorders of calcium homeostasis may decrease the adverse side effects associated with Vitamin D therapy alone.

Since RXR is known to form heterodimers with various members of the intracellular receptor super family, it can be expected that the synergistic response observed with use of RXR-selective ligands may be achieved with other receptors with which heterodimers are formed. These include PPARs, RARs, Vitamin D, thyroid hormone receptors, HNF4, the COUP family of receptors, as referenced above, and other as yet unidentified members of the intracellular super family of receptors.

As will be further discernible to those skilled in the art, the compounds disclosed above can be readily utilized in pharmacological applications where selective retinoid receptor activity is desired, and where it is desired to minimize cross reactivities with other related intracellular receptors. *In vivo* applications of the invention include administration of the disclosed compounds to mammalian subjects, and in particular to humans.

The compounds of the present invention are small molecules which are relatively fat soluble or lipophilic and enter the cell by passive diffusion across the plasma membrane. Consequently, these ligands are well suited for administration orally and by injection, as well as topically. Upon administration, these ligands can selectively activate retinoid X receptors, and thereby selectively modulate processes mediated by these receptors.

The pharmaceutical compositions of this invention are prepared in conventional dosage unit forms by incorporating an active compound of the invention, or a mixture of such compounds, with a nontoxic pharmaceutical carrier according to accepted procedures in a nontoxic amount sufficient to produce the desired pharmacodynamic activity in a mammalian and in particular a human subject. Preferably, the composition contains the active ingredient in an active, but nontoxic, amount selected from about 5 mg to about 500 mg of active ingredient per dosage unit. This quantity depends on the specific biological activity desired and the condition of the patient.

The pharmaceutical carrier or vehicle employed may be, for example, a solid or liquid. A variety of pharmaceutical forms can be employed. Thus, when using a solid carrier, the preparation can be plain milled, micronized in oil, tableted, placed in a hard gelatin or enteric-coated capsule in micronized powder or pellet form, or in the form of a troche, lozenge, or suppository. When using a liquid carrier, the preparation can be in the form of a liquid, such as an ampule, or as an aqueous or nonaqueous liquid suspension. For topical administration, the active ingredient may be formulated using bland, moisturizing bases, such as ointments or creams. Examples of suitable ointment bases are petrolatum, petrolatum plus volatile silicones, lanolin, and water in oil emulsions such as Eucerin (Beiersdorf). Examples of suitable cream bases are Nivea Cream (Beiersdorf), cold cream (USP), Purpose Cream (Johnson & Johnson) hydrophilic ointment (USP), and Lubriderm (Warner-Lambert).

The following examples provide illustrative pharmacological composition formulations:

<u>Example 36</u>

Hard gelatin capsules are prepared using the following ingredients:

| (mg/capsule) | Quantity |
|---|---|
| 3-methyl-TTNCB | 140 |
| Starch, dried | 100 |
| Magnesium stearate | 10 |
| Total | 250 mg |

The above ingredients are mixed and filled into hard gelatin capsules in 250 mg quantities.

Example 37

A tablet is prepared using the ingredients below:

| (mg/tablet) | Quantity |
|---|---|
| 3-methyl-TTNCB | 140 |
| Cellulose, microcrystalline | 200 |
| Silicon dioxide, fumed | 10 |
| Stearic acid | 10 |
| Total | 360 mg |

The components are blended and compressed to form tablets each weighing 360 mg.

Example 38

Tablets, each containing 60 mg of active ingredient, are made as follows:

| (mg/tablet) | Quantity |
|---|---|
| 3-methyl-TTNCB | 60 |
| Starch | 45 |
| Cellulose, microcrystalline | 35 |
| Polyvinylpyrrolidone (PVP) (as 10% solution in water) | 4 |
| Sodium carboxymethyl starch (SCMS) | 4.5 |
| Magnesium stearate | 0.5 |
| Talc | 1.0 |
| Total | 150 mg |

The active ingredient, starch, and cellulose are passed through a No. 45 mesh U.S. sieve and mixed thoroughly. The solution of PVP is mixed with the resultant powders, which are then passed through a No. 14 mesh U.S. sieve. The granules so produced are dried at 50°C and passed through a No. 18 mesh U.S. sieve. The SCMS, magnesium stearate, and talc, previously passed through a No. 60 mesh U.S. sieve, are then added to the granules which, after mixing, are compressed on a tablet machine to yield tablets each weighing 150 mg.

Example 39

Suppositories, each containing 225 mg of active ingredient, may be made as follows:

| | |
|---|---|
| 3-methyl-TTNCB | 225 mg |
| Saturated fatty acid glycerides | 2,000 mg |
| Total | 2,225 mg |

The active ingredient is passed through a No. 60 mesh U.S. sieve and suspended in the saturated fatty acid

glycerides previously melted using the minimum heat necessary. The mixture is then poured into a suppository mold of normal 2g capacity and allowed to cool.

Example 40

An intravenous formulation may be prepared as follows:

| | |
|---|---|
| 3-methyl-TTNCB | 100 mg |
| Isotonic saline | 1,000 ml |
| Glycerol | 100 ml |

The compound is dissolved in the glycerol and then the solution is slowly diluted with isotonic saline. The solution of the above ingredients is then administered intravenously at a rate of 1 ml per minute to a patient.

The compounds of this invention also have utility when labeled as ligands for use in assays to determine the presence of RXRs. They are particularly useful due to their ability to selectively bond to members of the RXR subfamily and can therefore be used to determine the presence of RXR isoforms in the presence of other related receptors.

Due to the selective specificity of the compounds of this invention for retinoid X receptors, these compounds can also be used to purify samples of retinoid X receptors *in vitro*. Such purification can be carried out by mixing samples containing retinoid X receptors with one of more of the bicyclic derivative compounds disclosed so that the compound (ligand) binds to the receptor, and then separating out the bound ligand/receptor combination by separation techniques which are known to those of skill in the art. These techniques include column separation, filtration, centrifugation, tagging and physical separation, and antibody complexing, among others.

While the preferred embodiments have been described and illustrated, various substitutions and modifications may be made thereto without departing from the scope of the invention. Accordingly, it is to be understood that the present invention has been described by way of illustration and not limitation.

**Claims**

1. A compound having the formula:

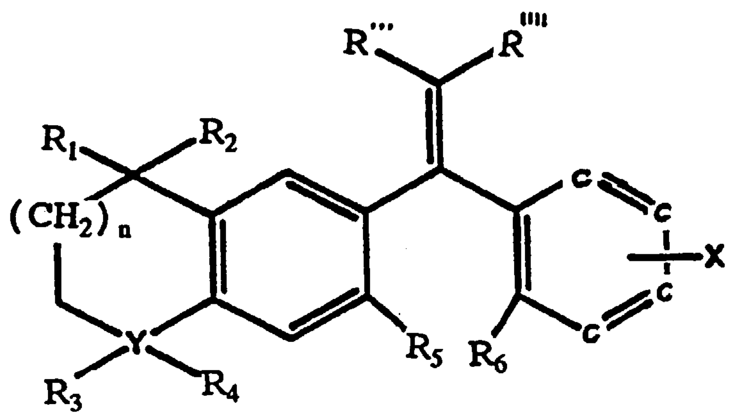

or

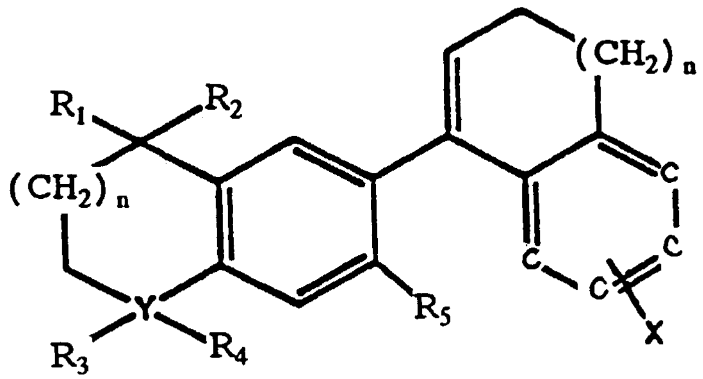

or

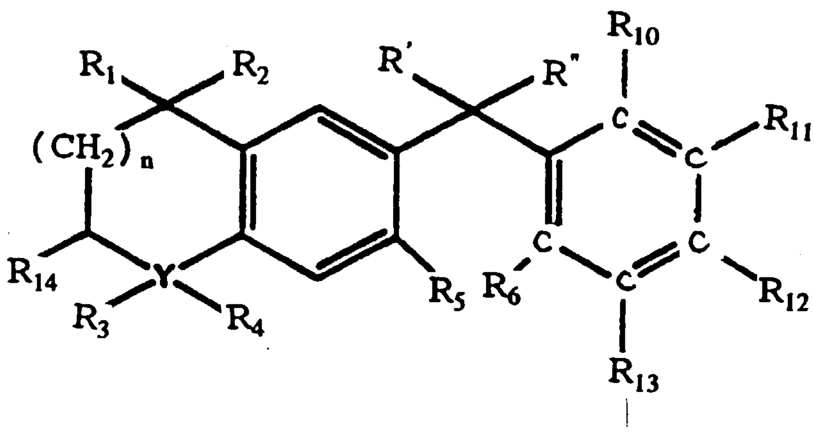

wherein

$R_1$ and $R_2$, each independently, represent hydrogen or lower alkyl or acyl having 1-4 carbon atoms;
Y represents C, O, S, N, CHOH, CO, SO, $SO_2$, or a pharmaceutically acceptable salt;

$R_3$ represents hydrogen or lower alkyl having 1-4 carbon atoms where Y is C or N;
$R_4$ represents hydrogen or lower alkyl having 1-4 carbon atoms where Y is C, but $R_4$ does not exist if Y is N, and neither $R_3$ or $R_4$ exist if Y is S, O, CHOH, CO, SO, or $SO_2$;
R' and R'' represent hydrogen, lower alkyl or acyl having 1-4 carbon atoms, OH, alkoxy having 1-4 carbon atoms, thiol or thio ether, or amino,
or R' and R'' taken together form an oxo (keto), methano, thioketo, epoxy, cyclopropyl, or cycloalkyl group and wherein the epoxy, cyclopropyl, and cycloalkyl groups can be substituted with lower alkyl having 1-4 carbons or halogen;
R''' and R'''' represent hydrogen, halogen, lower alkyl or acyl having 1-4 carbon atoms,
or R''' and R'''' taken together form a cycloalkyl group having 3-10 carbons, and wherein the cycloalkyl group can be substituted with lower alkyl having 1-4 carbons or halogen;
$R_5$ represents a lower alkyl having 1-4 carbons, halogen, nitro, $OR_7$, $SR_7$, $NR_7R_8$, or $(CF_2)_nCF_3$
$R_6$, $R_{10}$, $R_{11}$, $R_{12}$, $R_{13}$ each independently represent hydrogen, a lower alkyl having 1-4 carbons, halogen, nitro, $OR_7$, $SR_7$, $NR_7R_8$ or $(CF_2)_nCF_3$, with the proviso that one of $R_{11}$, $R_{12}$ or $R_{13}$ is X;
$R_7$ and $R_8$ each independently represent hydrogen or a lower alkyl having 1-6 carbons;
$R_{14}$ represents hydrogen, a lower alkyl having 1-4 carbons, cxc, hydroxy, acyl having 1-4 carbons, halogen, thiol, or thioketone;
X is COOH, tetrazole, $PO_3H$, $SO_3H$, CHO, $CH_2OH$, $CONH_2$, COSH, $COOR_9$, $COSR_9$, $CONHR_9$, or COOW where $R_9$ represents a lower alkyl having 1-4 carbons, phenyl, aromatic alkyl, or q-hydroxyphenyl, q-bromophenyl, q-chlorophenyl, q-fluorophenyl, or q-iodophenyl, where q=2-4, where W is a pharmaceutically acceptable salt, and where X can originate from any C except the 2 position on the ring;
n = 0-3.

**2.** A compound selected from the group consisting of 4-[(3,5,5,8,8-pentamethyl-5,6,7,8-tetrahydro-2-naphthyl)carbonyl]benzoic acid,

4-[1-(3,5,5,8,8-pentamethyl-5,6,7,8-tetrahydro-2-naphthyl)ethenyl]benzoic acid,
4-[1-(3-5,5,8,8-pentamethyl-5,6,7,8-tetrahydro-2-naphthyl)cyclopropyl]benzoic acid,
4-[1-(3,5,5,8,8-pentamethyl-5,6,7,8-tetrahydro-2-naphthyl)ethenyl]benzenetetrazole,
4-[1-(3,5,5,8,8-pentamethyl-5,6,7,8-tetrahydro-2-naphthyl)ethenyl]-N-(4-carboxyphenyl)benzamide,
4-[1-(3,5,5,8,8-pentamethyl-5,6,7,8-tetrahydro-2-naphthyl)ethenyl]-N-(3-carboxyphenyl)benzamide,
and
4-[1-(3,5,5,8,8-pentamethyl-5,6,7,8-tetrahydro-2-naphthyl)ethenyl]-N-(4-hydroxyphenyl)benzamide.

**3.** 4-[1-(3,5,5,8,8-Pentamethyl-5,6,7,8-tetrahydro-2-naphthyl)ethenyl]benzoic acid.

**4.** A pharmaceutical composition comprising in a pharmaceutically acceptable vehicle suitable for enteral, parenteral, or topical administration, one or more compound of claim 1.

**5.** A method for determining the presence of one or more Retinoid X Receptors comprising combining a compound of claim 1 with a sample containing one or more unknown receptors and determining whether said ligand binds to any receptor in said sample.

**6.** A method of purifying Retinoid X Receptors comprising combining a compound as set forth in claim 1 with a sample containing one or more said Retinoid X Receptors, allowing said compound to bind with Retinoid X Receptors, and separating out the bound combination of said compound and Retinoid X Receptor.

**7.** Use of a compound according to claim 1 in the preparation of a medicament for the modulation of in vivo lipid metabolism, skin-related processes, programmed cell death, malignant cell development, pre-malignant lesions, auto-immune diseases or fatty acid metabolism.

**8.** A compound as claimed in claim 1, 2 or 3, for administration to a mammalian subject to modulate a process mediated by one or more Retinoid X Receptors.

## Patentansprüche

**1.** Verbindung mit der Formel:

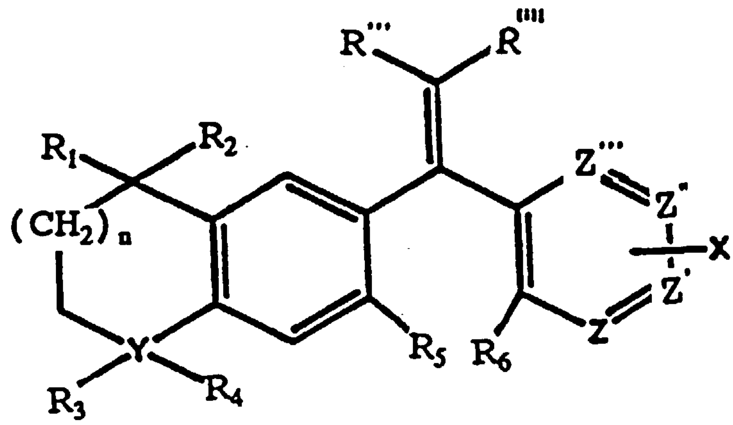

oder

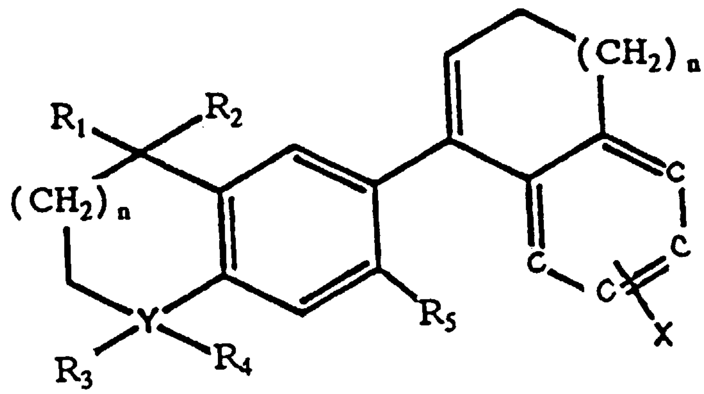

oder

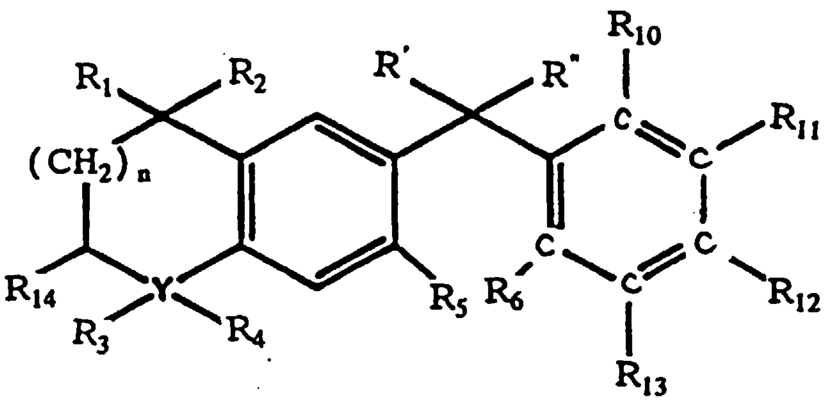

,

worin

$R_1$ und $R_2$, jeweils unabhängig, Wasserstoff oder Niederalkyl oder -acyl mit 1-4 Kohlenstoffatomen repräsentieren;
Y für C, O, S, N, CHOH, CO, SO, $SO_2$ oder ein pharmazeutisch annehmbares Salz steht;
$R_3$ Wasserstoff oder Niederalkyl mit 1-4 Kohlenstoffatomen repräsentiert, wobei Y C oder N ist;
$R_4$ Wasserstoff oder Niederalkyl mit 1-4 Kohlenstoffatomen repräsentiert, wobei Y C ist, aber $R_4$ nicht existiert, wenn Y N ist, und weder $R_3$ noch $R_4$ existieren, wenn Y S, O, CHOH, CO, SO oder $SO_2$ ist;
R' und R" Wasserstoff, Niederalkyl oder -acyl mit 1-4 Kohlenstoffatomen, OH, Alkoxy mit 1-4 Kohlenstoffatomen, Thiol oder Thioether oder Amino repräsentieren,
oder R' und R" zusammengenommen eine Oxo- (Keto-), Methano-, Thioketo-, Epoxy-, Cyclopropyl- oder Cycloalkylgruppe bilden, und wobei die Epoxy-, Cyclopropyl- und Cycloalkylgruppen mit Niederalkyl mit 1-4

Kohlenstoffatomen oder Halogen substitiuiert sein können;

R''' und R'''' Wasserstoff, Halogen, Niederalkyl oder -acyl mit 1-4 Kohlenstoffatomen repräsentieren, oder R''' und R'''' zusammengenommen eine Cycloalkylgruppe mit 3-10 Kohlenstoffatomen bilden, und wobei die Cycloalkylgruppe mit Niederalkyl mit 1-4 Kohlenstoffatomen oder Halogen substitiuiert sein kann;

$R_5$ ein Niederalkyl mit 1-4 Kohlenstoffatomen, Halogen, Nitro, $OR_7$, $SR_7$, $NR_7R_8$ oder $(CF_2)_nCF_3$ repräsentiert, $R_6$, $R_{10}$, $R_{11}$, $R_{12}$, $R_{13}$ jeweils unabhängig Wasserstoff, ein Niederalkyl mit 1-4 Kohlenstoffatomen, Halogen, Nitro, $OR_7$, $SR_7$, $NR_7R_8$, oder $(CF_2)_nCF_3$ repräsentieren, mit der Maßgabe, daß eines von $R_{11}$, $R_{12}$ oder $R_{13}$ X ist;

$R_7$ und $R_8$ jeweils unabhängig Wasserstoff oder ein Niederalkyl mit 1-6 Kohlenstoffatomen repräsentieren;

$R_{14}$ Wasserstoff, ein Niederalkyl mit 1-4 Kohlenstoffatomen, Oxo, Hydroxy, Acyl mit 1-4 Kohlenstoffatomen, Halogen, Thiol oder Thioketon repräsentiert;

X COOH, Tetrazol, $PO_3H$, $SO_3H$, CHO, $CH_2OH$, $CONH_2$, COSH, $COOR_9$, $COSR_9$, $CONHR_9$ oder COOW ist, wobei $R_9$ ein Niederalkyl mit 1-4 Kohlenstoffatomen, Phenyl, aromatisches Alkyl, oder q-Hydroxyphenyl, q-Bromphenyl, q-Chlorphenyl, q-Fluorphenyl oder q-Iodphenyl repräsentiert, wobei q = 2-4 ist, wobei W ein pharmazeutisch annehmbares Salz ist, und wobei X von jedwedem C, außer der Position 2, auf dem Ring ausgehen kann;

n = 0-3 ist.

2. Verbindung, gewählt aus der Gruppe, bestehend aus

4-[(3,5,5,8,8-Pentamethyl-5,6,7,8-tetrahydro-2-naphthyl)carbonyl]benzoesäure,
4-[1-(3,5,5,8,8-Pentamethyl-5,6,7,8-tetrahydro-2-naphthyl)ethenyl]benzoesäure,
4-[1-(3,5,5,8,8-Pentamethyl-5,6,7,8-tetrahydro-2-naphthyl)cyclopropyl]benzoesäure,
4-[1-(3,5,5,8,8-Pentamethyl-5,6,7,8-tetrahydro-2-naphthyl)ethenyl]benzoltetrazol,
4-[1-(3,5,5,8,8-Pentamethyl-5,6,7,8-tetrahydro-2-naphthyl)ethenyl]-N-(4-carboxyphenyl)benzamid,
4-[1-(3,5,5,8,8-Pentamethyl-5,6,7,8-tetrahydro-2-naphthyl)ethenyl]-N-(3-carboxyphenyl)benzamid,
4-[1-(3,5,5,8,8-Pentamethyl-5,6,7,8-tetrahydro-2-naphthyl)ethenyl]-N-(4-hydroxyphenyl)benzamid.

3. 4-[1-(3,5,5,8,8-Pentamethyl-5,6,7,8-tetrahydro-2-naphthyl)ethenyl]benzoesäure

4. Pharmazeutische Zusammensetzung, umfassend in einem pharmazeutisch annehmbaren Vehikel, geeignet für enterale, parenterale oder topische Verabreichung, eine oder mehrere Verbindung(en) von Anspruch 1.

5. Verfahren zur Bestimmung des Vorhandenseins eines oder mehrerer Retinoid-X-Rezeptoren, umfassend das Vereinigen einer Verbindung von Anspruch 1 mit einer Probe, enthaltend einen oder mehrere unbekannte Rezeptoren, und das Bestimmen, ob der Ligand an irgendeinen Rezeptor in der Probe bindet.

6. Verfahren zur Reinigung von Retinoid-X-Rezeptoren, umfassend das Vereinigen einer Verbindung, wie dargestellt in Anspruch 1, mit einer Probe, enthaltend einen oder mehrere besagte Retinoid-X-Rezeptoren, das Binden lassen der Verbindung mit Retinoid-X-Rezeptoren und das Abtrennen der gebundenen Kombination der Verbindung und des Retinoid-X-Rezeptors.

7. Verwendung einer Verbindung gemäß Anspruch 1 bei der Herstellung eines Medikamentes für die Modulation von in vivo-Lipidstoffwechsel, von mit der Haut zusammenhängenden Vorgängen, von programmiertem Zelltod, maligner Zellentwicklung, prämalignen Läsionen, Autoimmunkrankheiten oder des Fettsäurestoffwechsels.

8. Verbindung, wie beansprucht in Anspruch 1, 2 oder 3 zur Verabreichung an ein Säugersubjekt, um einen von einem oder mehreren Retinoid-X-Rezeptoren vermittelten Vorgang zu modulieren.

**Revendications**

1. Composé présentant la formule :

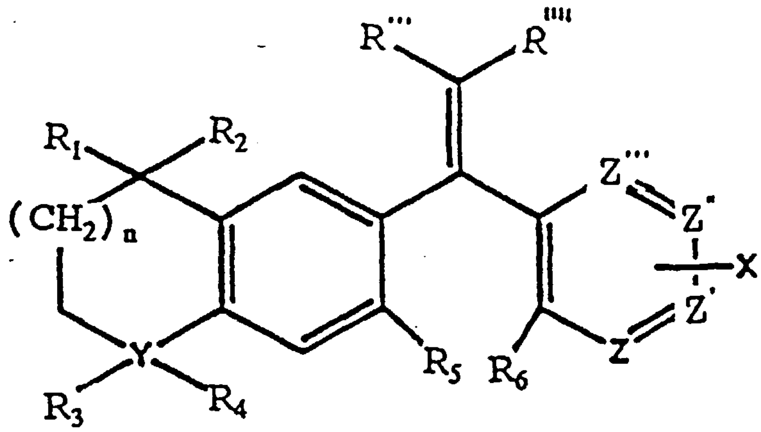

ou

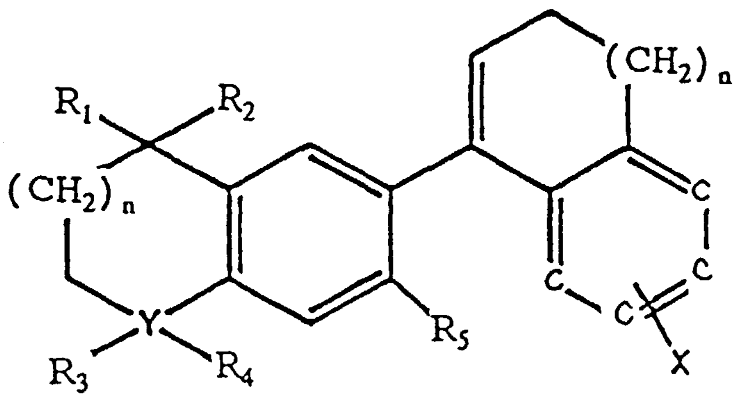

ou

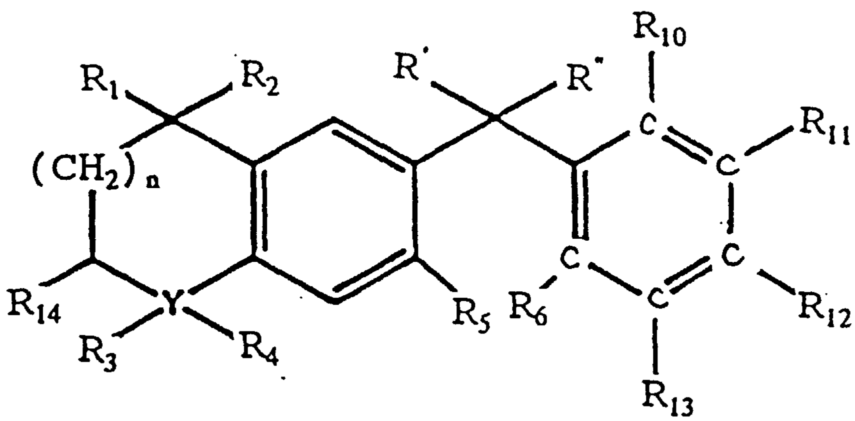

dans laquelle

$R_1$ et $R_2$ représentent, chacun indépendamment, un hydrogène ou un alkyle ou acyle inférieur ayant 1 à 4 atomes de carbone ;

Y représente C, O, S, N, CHOH, CO, SO, $SO_2$, ou un sel pharmaceutiquement acceptable ;
$R_3$ représente un hydrogène ou un alkyle inférieur ayant 1 à 4 atomes de carbone dans lequel Y est C ou N ;
$R_4$ représente un hydrogène ou un alkyle inférieur ayant 1 à 4 atomes de carbone dans lequel Y est C, mais R4 n'existe pas si Y est N, et ni $R_3$ ni $R_4$ n'existe si Y est S, O, CHOH, CO, SO, ou $SO_2$ ;
R' et R'' représentent un hydrogène, un alkyle ou acyle inférieur ayant 1 à 4 atomes de carbone, OH, un alcoxy ayant 1 à 4 atomes de carbone, un thiol ou un thioéther, ou un amino,
ou R' et R'' pris ensemble forment un groupe oxo (céto), méthano, thiocéto, époxy, cyclopropyle, ou cycloalkyle et dans lequel les groupes époxy, cyclopropyle, et cycloalkyle peuvent être substitués par un alkyle inférieur ayant 1 à 4 carbones ou un halogène ;
R''' et R'''' représentent un hydrogène, un halogène, un alkyle ou acyle inférieur ayant 1 à 4 atomes de carbone, ou R''' et R'''' pris ensemble forment un groupe cycloalkyle ayant 3 à 10 carbones, et dans lequel le groupe cycloalkyle peut être substitué par un alkyle inférieur ayant 1 à 4 carbones ou un halogène ;
$R_5$ représente un alkyle inférieur ayant 1 à 4 carbones, un halogène, un nitro, $OR_7$, $SR_7$, $NR_7R_8$, ou $(CF_2)_nCF_3$ ;
$R_6$, $R_{10}$, $R_{11}$, $R_{12}$, $R_{13}$ représentent chacun indépendamment un hydrogène, un alkyle inférieur ayant 1 à 4 carbones, un halogène, un nitro, $OR_7$, $SR_7$, $NR_7R_8$ ou $(CF_2)_nCF_3$ à la condition qu'un des $R_{11}$, $R_{12}$ ou $R_{13}$ soit X ;
$R_7$ et $R_8$ représentent chacun indépendamment un hydrogène ou un alkyle inférieur ayant 1 à 6 carbones ;
$R_{14}$ représente un hydrogène, un alkyle inférieur ayant 1 à 4 carbones, un oxo, un hydroxy, un acyle ayant 1 à 4 carbones, un halogène, un thiol, ou une thiocétone ;
X est COOH, un tétrazole, $PO_3H$, $SO_3H$, CHO, $CH_2OH$, $CONH_2$, COSH, $COOR_9$, $COSR_9$, $CONHR_9$, ou COOW dans lequel $R_9$ représente un alkyle inférieur ayant 1 à 4 carbones, un phényle, un alkyle aromatique, ou un q-hydroxyphényle, q-bromophényle, q-chlorophényle, q-fluorophényle, ou q-iodophényle, dans lequel q = 2 à 4, dans lequel W est un sel pharmaceutiquement acceptable, et dans lequel X peut provenir d'un C quelconque excepté celui en position 2 dans le cycle ;
n = 0 à 3.

2. Composé choisi dans le groupe constitué par l'acide 4-[ (3,5,5,8,8-pentaméthyl-5,6,7,8-tétrahydro-2-naphtyl)carbonyl]benzoïque,

l'acide 4-[1-(3,5,5,8,8-pentaméthyl-5,6,7,8-tétrahydro-2-naphtyl)éthényl]benzoïque,
l'acide 4-[1-(3,5,5,8,8-pentaméthyl-5,6,7,8-tétrahydro-2-naphtyl)cyclopropyl]benzoïque,
le 4-[1-(3,5,5,8,8-pentaméthyl-5,6,7,8-tétrahydro-2-naphtyl)éthényl]benzènetétrazole,
le 4-[1-(3,5,5,8,8-pentaméthyl-5,6,7,8-tétrahydro-2-naphtyl)éthényl]-N-(4-carboxyphényl)benzamide,
le 4-[1-(3,5,5,8,8-pentaméthyl-5,6,7,8-tétrahydro-2-naphtyl)éthényl]-N-(3-carboxyphényl)benzamide, et
le 4-[1-(3,5,5,8,8-pentaméthyl-5,6,7,8-tétrahydro-2-naphtyl)éthényl]-N-(4-hydroxyphényl)benzamide.

3. Acide 4-[1-(3,5,5,8,8-pentaméthyl-5,6,7,8-tétrahydro-2-naphtyl)éthényl]benzoïque.

4. Composition pharmaceutique comprenant dans un véhicule pharmaceutiquement acceptable convenant pour une administration entérale, parentérale, ou topique, un ou plusieurs composés selon la revendication 1.

5. Méthode pour la détermination de la présence d'un ou plusieurs récepteurs X de rétinoides comprenant les étapes consistant à combiner un composé selon la revendication 1 avec un échantillon contenant un ou plusieurs récepteurs inconnus et à déterminer si ledit ligand se lie à un récepteur quelconque dans ledit échantillon.

6. Méthode de purification de récepteurs X de rétinoides comprenant les étapes consistant à combiner un composé tel que décrit à la revendication 1 avec un échantillon contenant un ou plusieurs desdits récepteurs X de rétinoïdes, à permettre audit composé de se lier aux récepteurs X de rétinoïdes, et à séparer la combinaison liée desdits composé et récepteur X de rétinoïdes.

7. Utilisation d'un composé selon la revendication 1 dans la préparation d'un médicament destiné à moduler in vivo le métabolisme des lipides, les processus faisant intervenir la peau, la mort cellulaire programmée, le développement de cellules malignes, les lésions pré-malignes, les maladies auto-immunes ou le métabolisme des acides gras.

8. Composé tel que revendiqué à la revendication 1, 2 ou 3, destiné à une administration à un sujet mammifère afin

de moduler un processus sous la médiation d'un ou plusieurs récepteurs X de rétinoïdes.

FIG. 1.

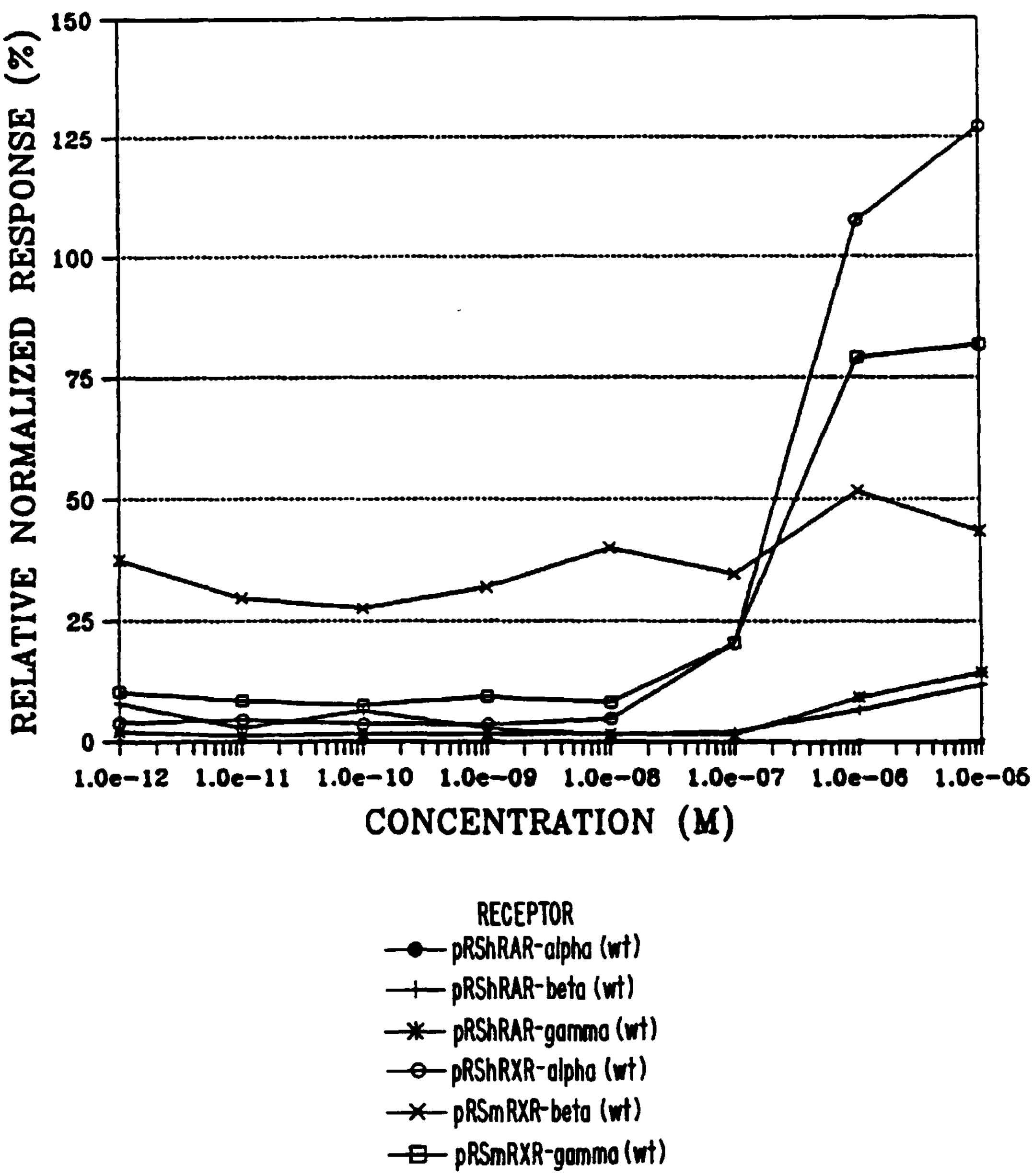
150
125
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 2.

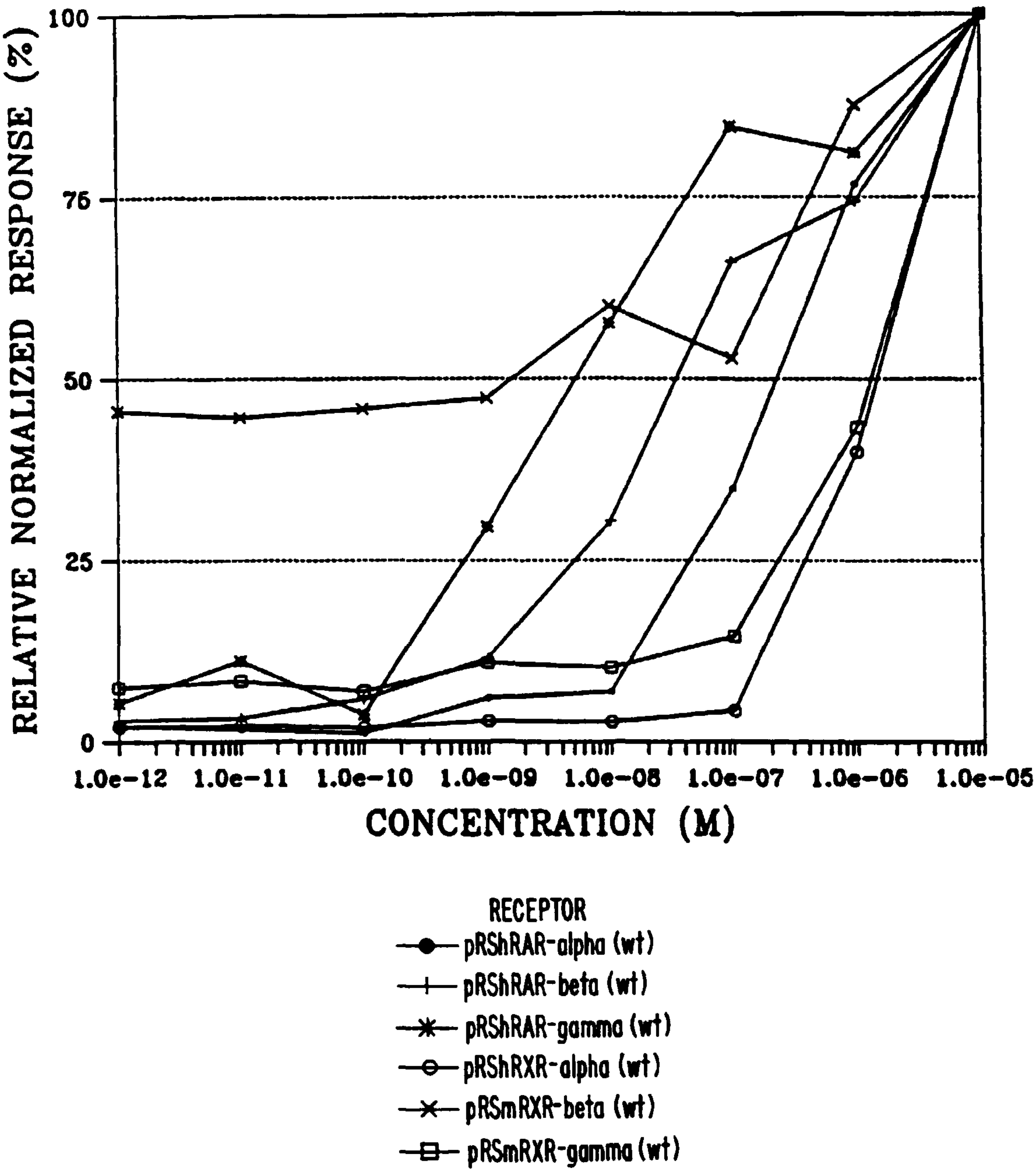
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 3.

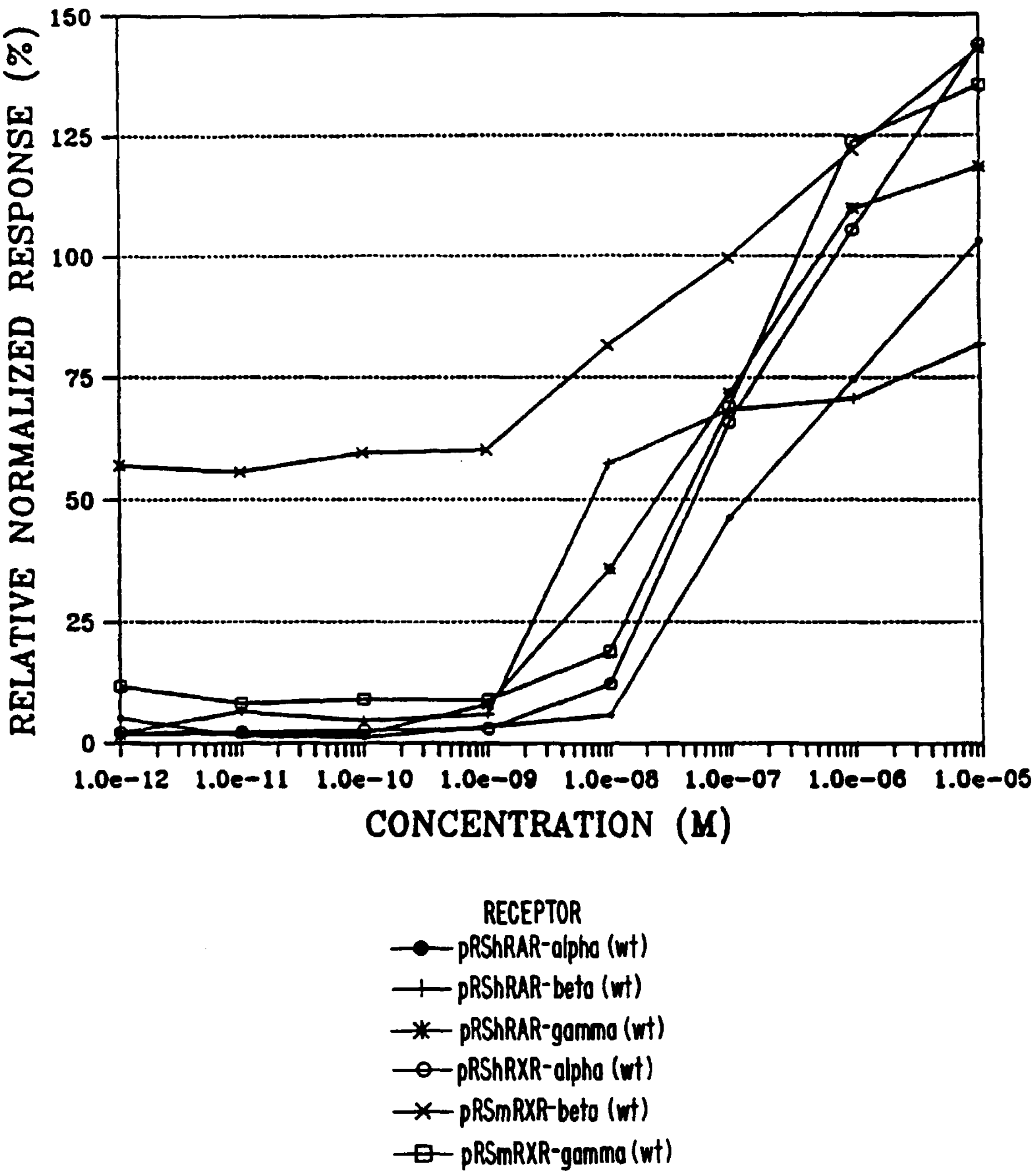
150
125
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 4.

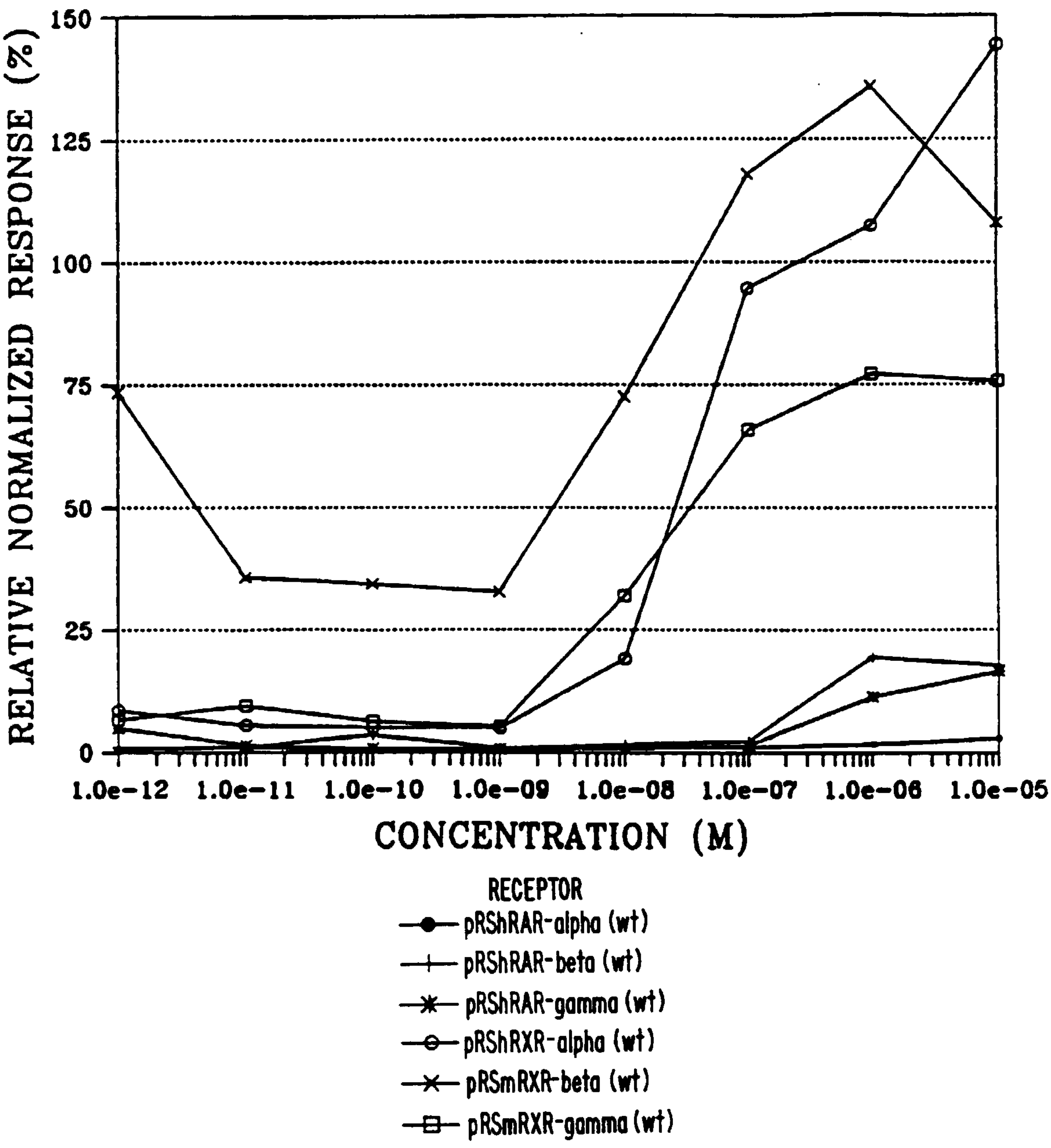
RELATIVE NORMALIZED RESPONSE (%)
0
25
50
75
100
125
150
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 5.

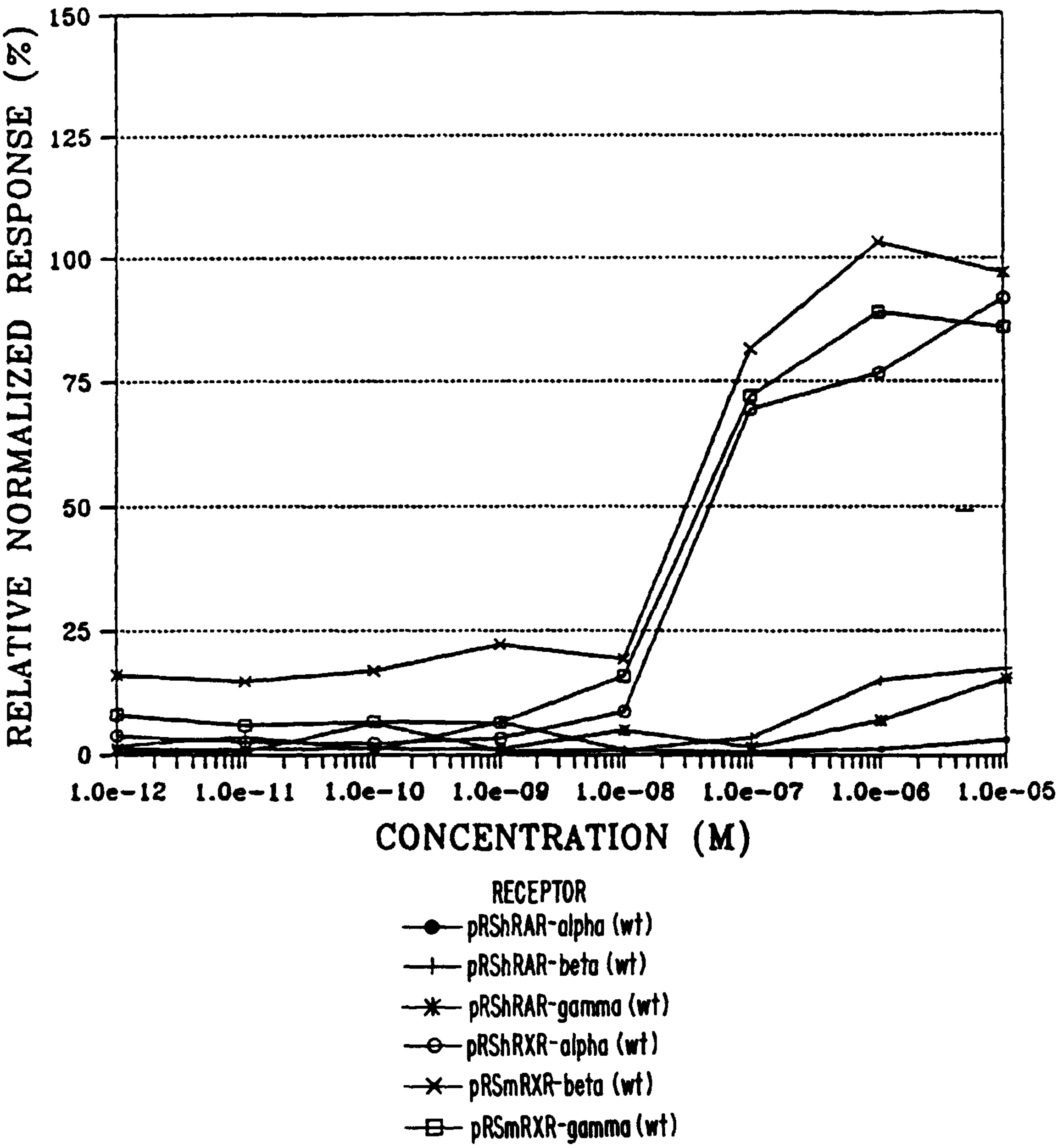
150
125
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 6.

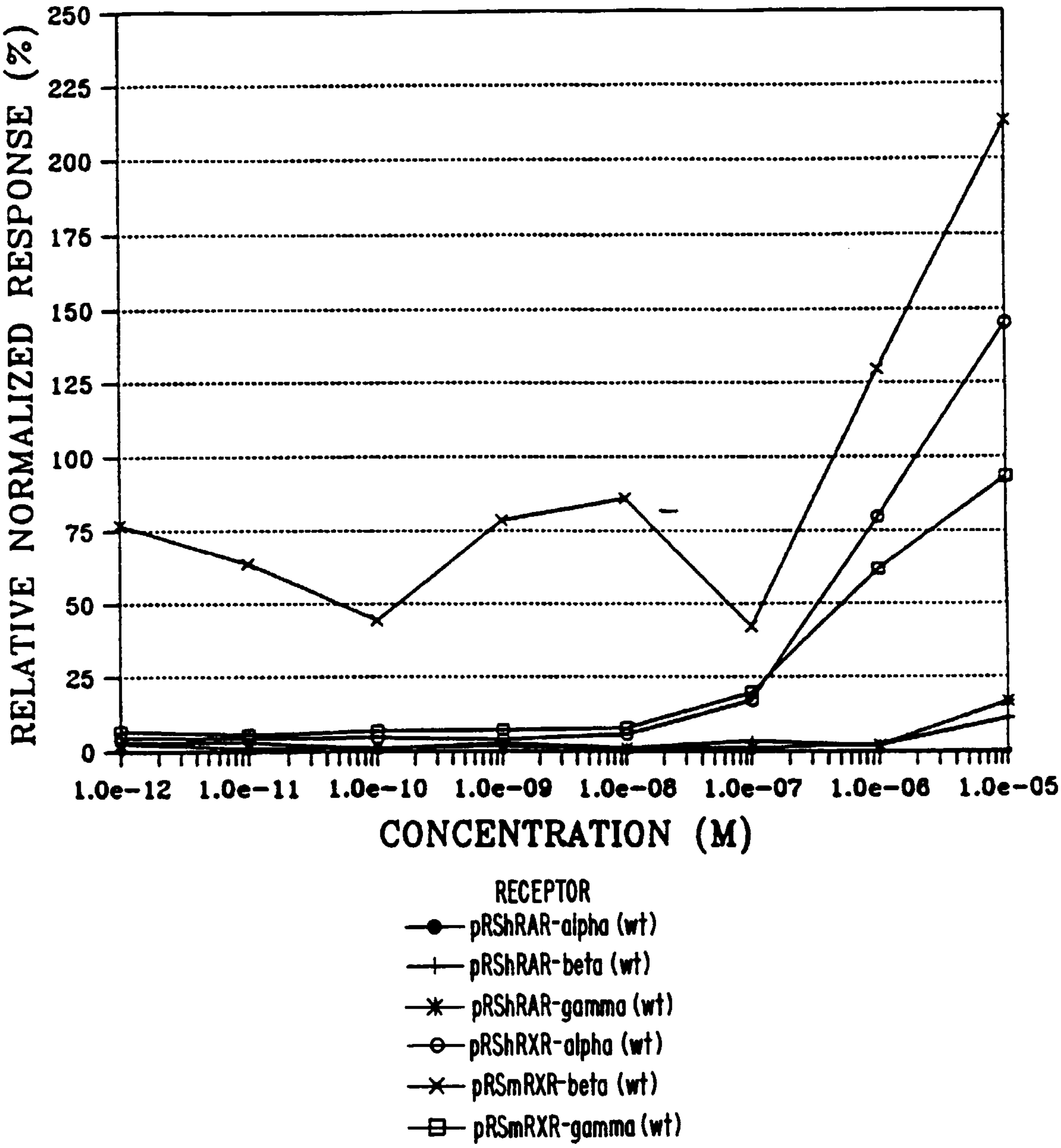

RELATIVE NORMALIZED RESPONSE (%)
250
225
200
175
150
125
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 7.

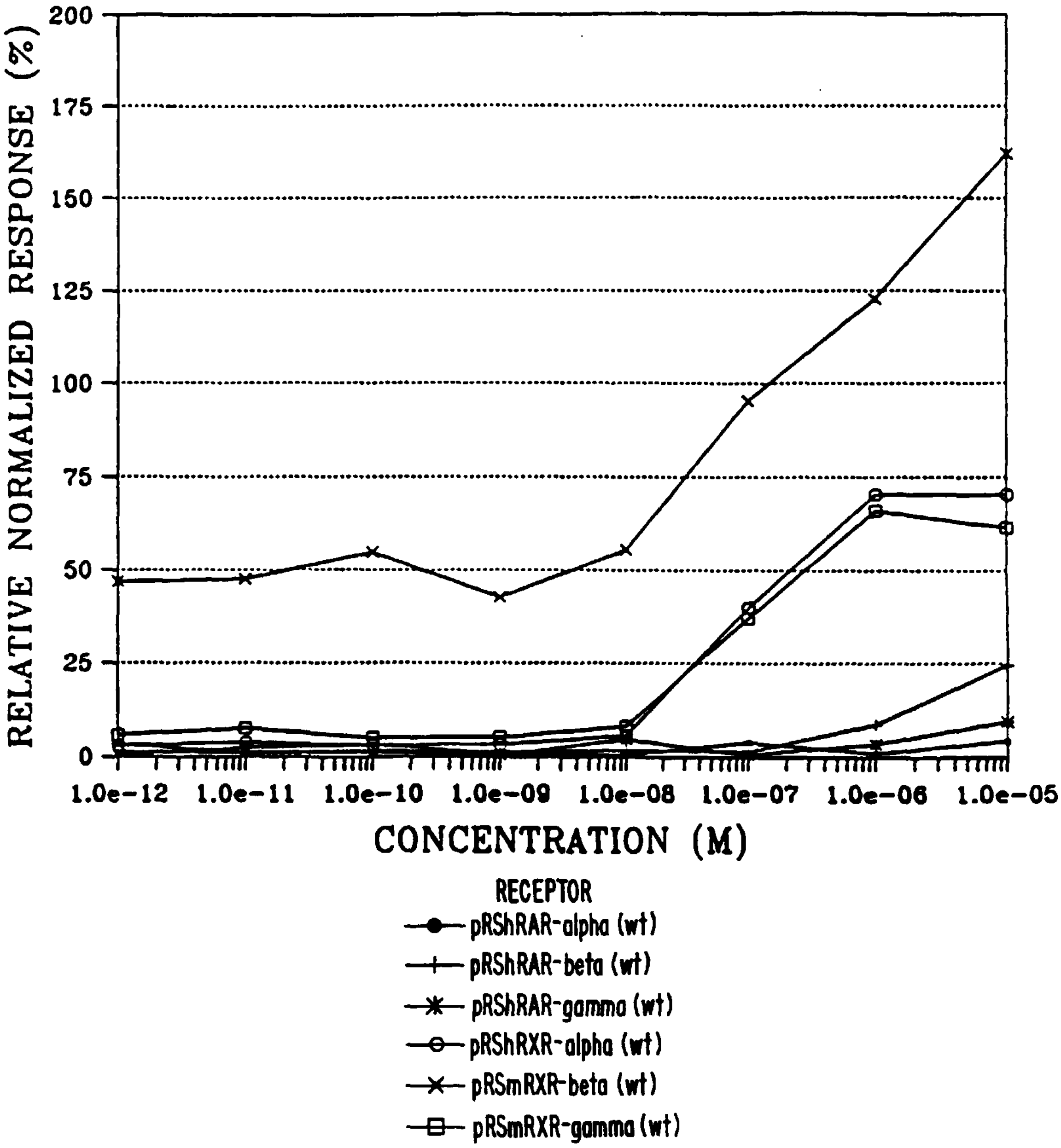
RELATIVE NORMALIZED RESPONSE (%)
200
175
150
125
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 8.

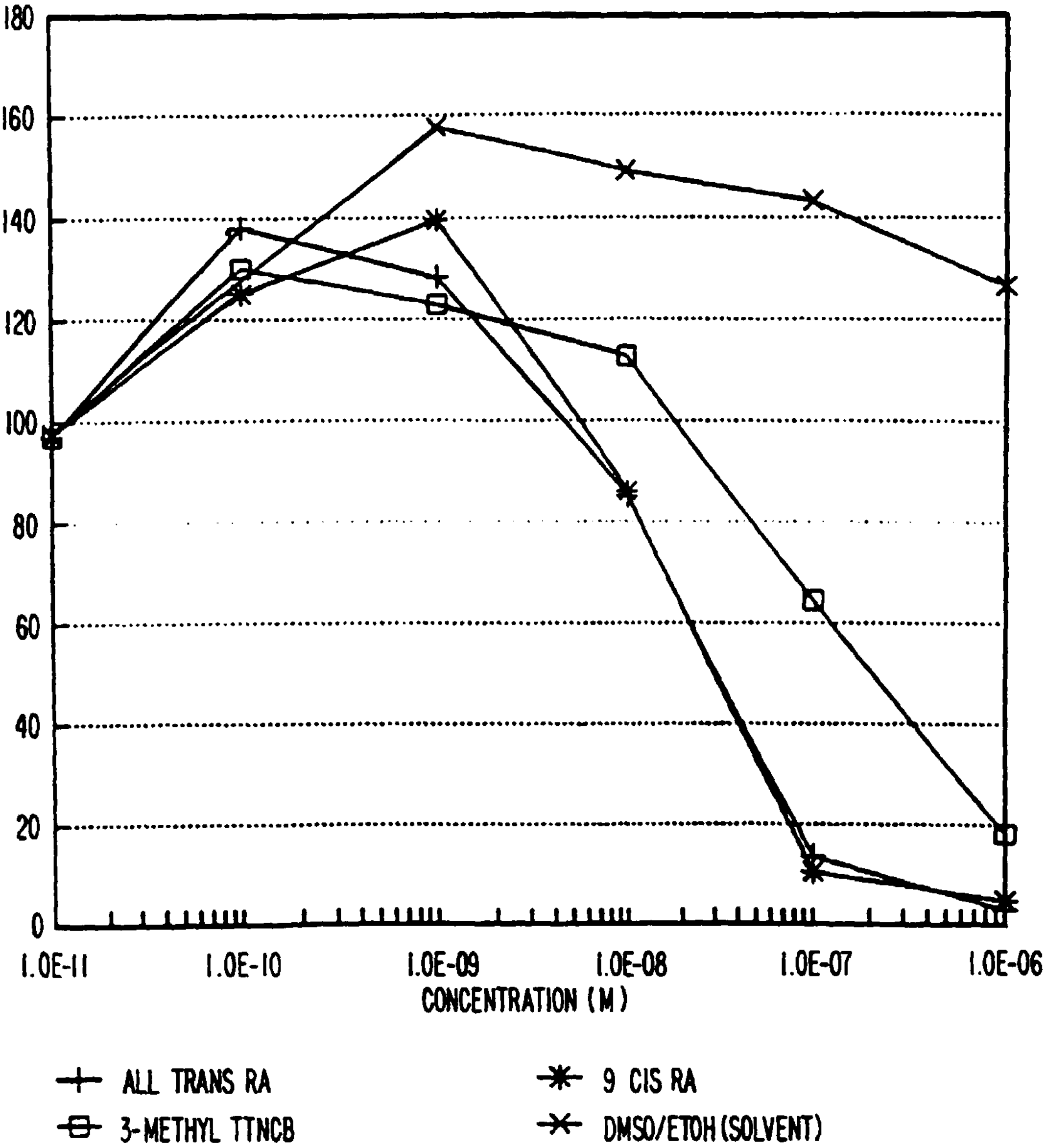
180
160
140
120
100
80
60
40
20
0
1.0E-11
1.0E-10
1.0E-09
1.0E-08
1.0E-07
1.0E-06
CONCENTRATION (M)
ALL TRANS RA
3-METHYL TTNCB
9 CIS RA
DMSO/ETOH (SOLVENT)

FIG. 9

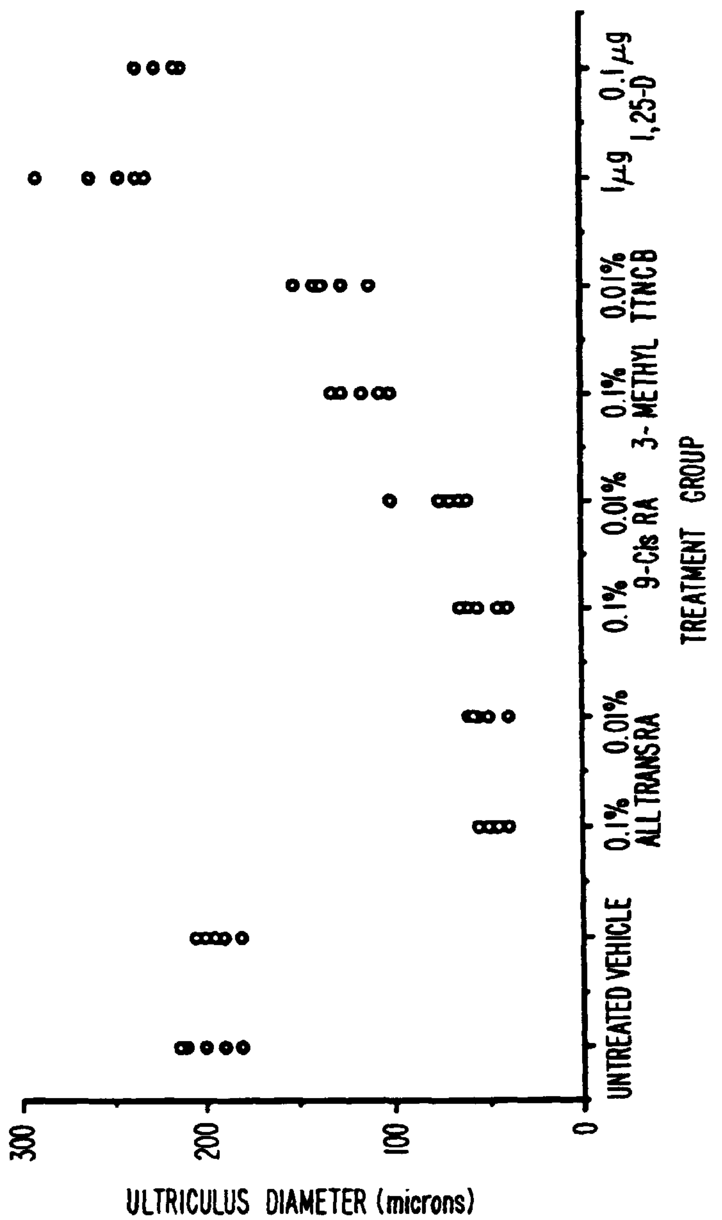
ULTRICULUS DIAMETER (microns)
300
200
100
0
UNTREATED
VEHICLE
0.1%
0.01%
ALL TRANS RA
0.1%
0.01%
9-Cis RA
0.1%
0.01%
3- METHYL TTNCB
1μg
0.1μg
1,25-D
TREATMENT GROUP

FIG. 10.

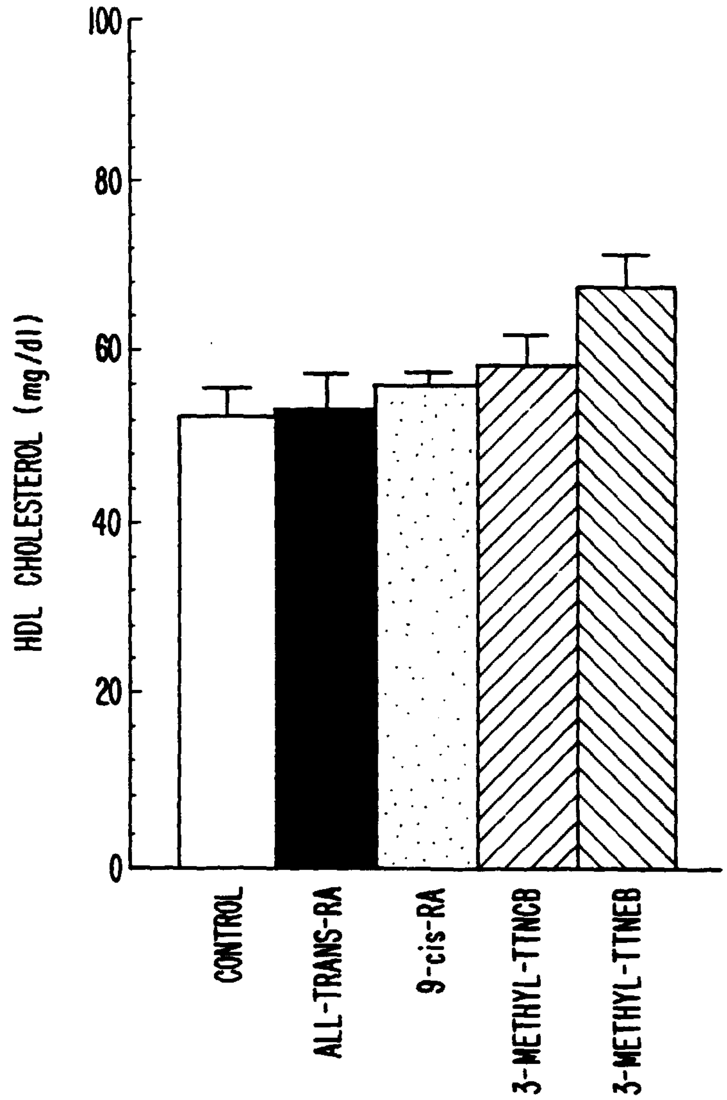
100
80
60
40
20
0
HDL CHOLESTEROL (mg/dl)
CONTROL
ALL-TRANS-RA
9-cis-RA
3-METHYL-TTNCB
3-METHYL-TTNEB

FIG. 11.

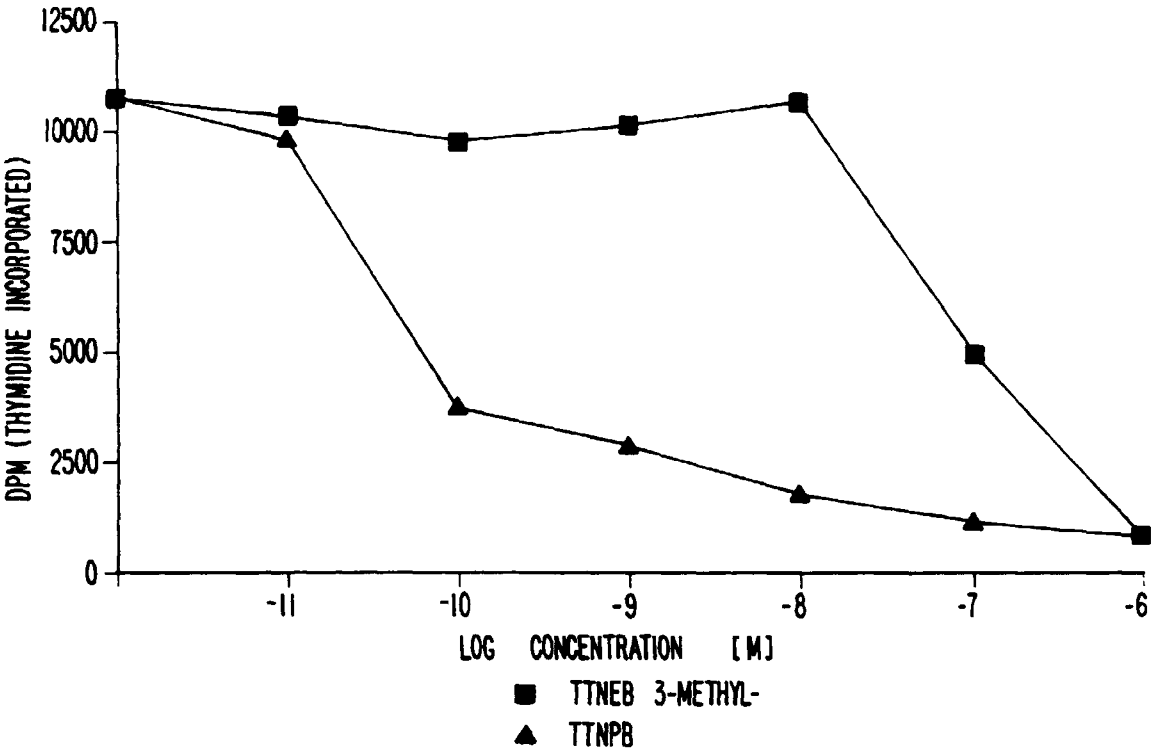
12500
10000
7500
5000
2500
0
DPM (THYMIDINE INCORPORATED)
-11
-10
-9
-8
-7
-6
LOG CONCENTRATION [M]
TTNEB 3-METHYL-
TTNPB

FIG. 12.

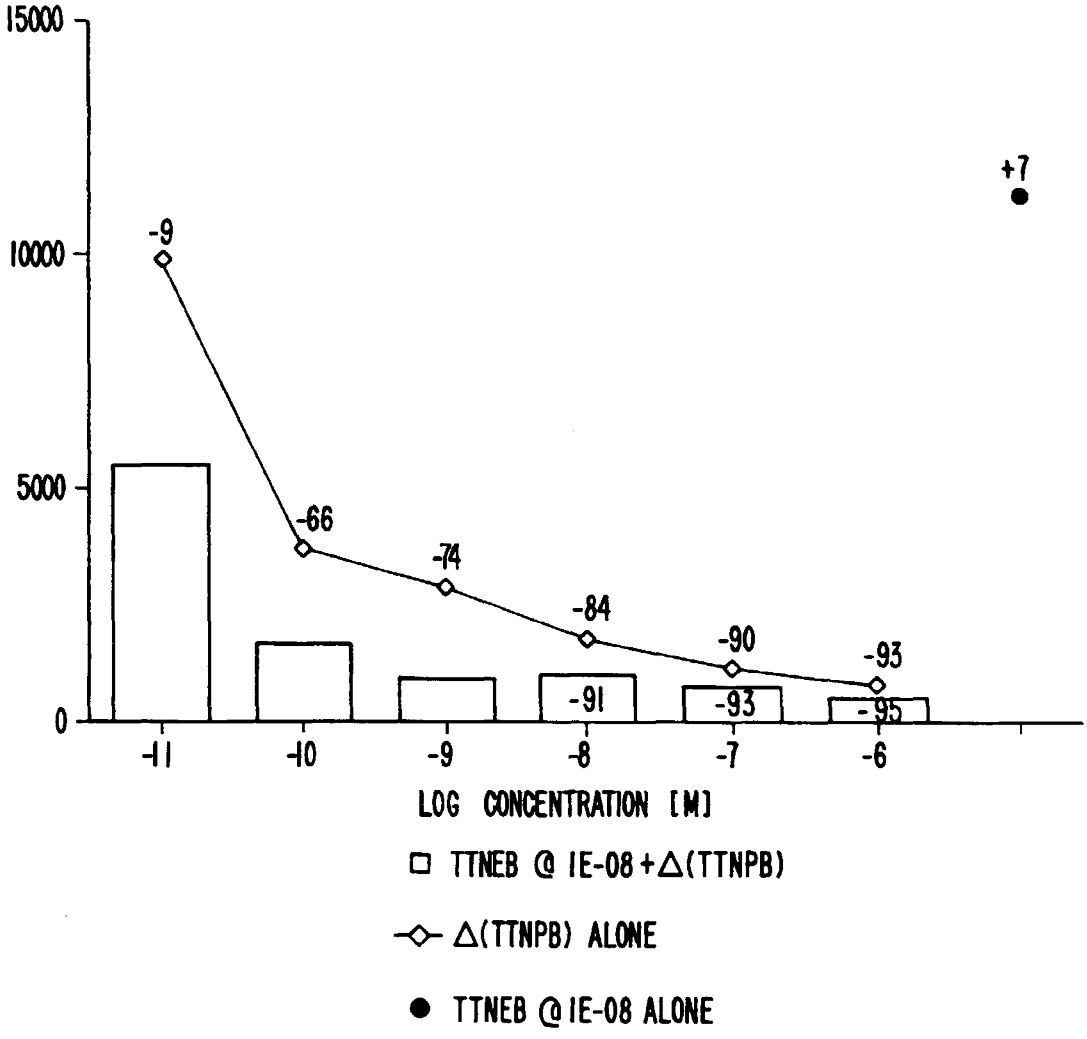
15000
10000
5000
0
+7
-9
-66
-74
-84
-90
-93
-91
-93
-95
-11
-10
-9
-8
-7
-6
LOG CONCENTRATION [M]
TTNEB @ 1E-08+Δ(TTNPB)
Δ(TTNPB) ALONE
TTNEB @ 1E-08 ALONE